(19) Europäisches Patentamt
European Patent Office
Office européen des brevets

(11) Veröffentlichungsnummer: 0 287 833 B1

# (12) EUROPÄISCHE PATENTSCHRIFT

(45) Veröffentlichungstag der Patentschrift: 12.09.90

(51) Int. Cl.⁵: G03C 7/305, C07D 403/06, C07D 239/88, C07D 249/02

(21) Anmeldenummer: 88104711.2

(22) Anmeldetag: 24.03.88

## (54) Farbfotografisches Aufzeichnungsmaterial mit einem Kuppler, der eine fotografisch wirksame Verbindung freisetzt.

(30) Priorität: 04.04.87 DE 3711418

(43) Veröffentlichungstag der Anmeldung: 26.10.88 Patentblatt 88/43

(45) Bekanntmachung des Hinweises auf die Patenterteilung: 12.09.90 Patentblatt 90/37

(84) Benannte Vertragsstaaten: BE DE FR GB

(56) Entgegenhaltungen: DE-A- 3 427 235

(73) Patentinhaber: Agfa-Gevaert AG, D-5090 Leverkusen 1(DE)

(72) Erfinder: Bergthaller, Peter, Dr., Leuchter Gemark 5 A, D-5060 Bergisch-Gladbach 2(DE)
Erfinder: Stolzenburg, Rudolf, Weissenstein 94, D-4018 Langenfeld(DE)
Erfinder: Hübner, Dirk, Dr., Andreas-Gryphius-Strasse 11, D-5000 Köln 80(DE)

EP 0 287 833 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein farbfotografisches Aufzeichnungsmaterial mit mindestens einer lichtempfindlichen Silberhalogenidemulsionsschicht, die einen Kuppler enthält, der bei Farbentwicklung eine fotografisch wirksame Gruppe, z.B. einen Entwicklungsinhibitor freisetzt.

Es ist bekannt, die chromogene Entwicklung in Gegenwart von Verbindungen durchzuführen, die bei der Entwicklung bildmäßig diffusionsfähige Substanzen freisetzen, die eine bestimmte Wirkung entfalten, beispielsweise die Entwicklung von Silberhalogenid zu beeinflussen vermögen. Falls diese Wirkung darin besteht, daß die weitere Entwicklung inhibiert wird, werden derartige Verbindungen als sogenannte DIR-Verbindungen (DIR = development inhibitor releasing) bezeichnet. Bei den DIR-Verbindungen kann es sich um solche handeln, die unter Abspaltung eines Inhibitorrestes mit dem Oxida tionsprodukt eines Farbentwicklers zu einem Farbstoff reagieren (DIR-Kuppler), oder um solche, die den Inhibitor freisetzen ohne gleichzeitig einen Farbstoff zu bilden. Letztere werden auch als DIR-Verbindungen im engeren Sinne bezeichnet.

DIR-Kuppler sind beispielsweise bekannt aus US-A-3 148 062, US-A-3 227 554, US-A-3 615 506, US-A-3 617 291 und DE-A-2 414 006.

Bei den diffusionsfähigen, fotografisch wirksamen Verbindungen, die bei der Entwicklung freigesetzt werden, kann es sich aber beispielsweise auch um einen Farbstoff, einen Kuppler, ein Härtungsmittel, ein Silberhalogenidlösungsmittel, ein Verschleierungsmittel, einen Entwicklungsbeschleuniger, eine Entwicklerverbindung, einen Bleichinhibitor, einen Bleichbeschleuniger, ein Beizmittel oder einen Sensibilisator handeln.

Bei freigesetzten Entwicklungsinhibitoren handelt es sich in der Regl um heterocyclische Mercaptoverbindungen oder um Derivate des Benzotriazols. Hinsichtlich der im wesentlichen farblos kuppelnden DIR-Verbindungen sei beispielsweise verwiesen auf US-A-3 632 345, DE-A-23 59 295 und DE-A-25 40 959. Durch Anwendung von DIR-Verbindungen kann eine Vielzahl von fotografischen, die Bildqualität beeinflussenden Effekten bewirkt werden. Solche Effekte sind beispielsweise die Erniedrigung der Gradation, die Erzielung eines feineren Farbkorns, die Verbesserung der Schärfe durch den sogenannten Kanteneffekt und die Verbesserung der Farbreinheit und der Farbbrillanz durch sogenannte Interimageeffekte. Zu verweisen ist beispielsweise auf die Publikation "Development-Inhibitor-Releasing (DIR) Couplers in Color Photography" von C.R. Barr, J.R. Thirtle und P.W. Vittum, Photographie Science and Engineering 13, 74 (1969).

Die farblos kuppelnden DIR-Verbindungen haben vor den farbig kuppelnden DIR-Kupplern den Vorteil, daß sie universell einsetzbar sind, so daß die gleiche Verbindung ohne Rücksicht auf die zu erzeugende Farbe in allen lichtempfindlichen Schichten eines farbfotografischen Aufzeichnungsmaterials verwendet werden kann. DIR-Kuppler können dagegen wegen der aus ihnen erzeugten Farbe meist nur in einem Teil der lichtempfindlichen Schichten verwendet werden, falls nicht die auf sie zurückzuführende Farbnebendichte in den andren Schichten tolerierbar ist. Diesem Vorteil der DIR-Verbindungen steht als Nachteil gegenüber, daß sie im allgemeinen weniger reaktiv sind als die DIR-Kuppler. In der Praxis hat man sich daher meist darauf beschränkt, DIR-Kuppler zu verwenden und zwar notfalls zwei oder mehrere verschiedene im gleichen Aufzeichnungsmaterial, wobei den unterschiedlich spektral sensibilisierten Schichten verschiedene DIR-Kuppler nach Maßgabe der aus den letzteren erzeugten Farbe zuzuordnen waren.

Es ist normalerweise wichtig, daß die fotografisch wirksame Verbindung bei Entwicklung rasch aus dem Kuppler freigesetzt wird, und zwar insbesondere dann, wenn die fotografisch wirksame Verbindung den weiteren Verlauf der Entwicklung beeinflussen soll. Es ist daher sehr erwünscht, wenn die betreffenden Kuppler sehr aktiv sind. Hierbei kommt der an die Kupplungsstelle des Kupplers gebundene Gruppe der fotografisch wirksamen Verbindung als sogenannte Fluchtgruppe besondere Bedeutung zu.

Der Erfindung liegt die Aufgabe zugrunde, ein farbfotografisches Aufzeichnungsmaterial anzugeben, das Kuppler enthält, aus denen bei der Entwicklung ein an die Kupplungsstelle gebundener Rest als fotografisch wirksame Verbindung freigesetzt wird.

Gegenstand der Erfindung ist ein farbfotografisches Aufzeichnungsmaterial mit mindestens einer lichtempfindlichen Silberhalogenidemulsionsschicht und einem dieser zugeordneten Kuppler, der eine fotografisch wirksame Verbindung freizusetzen vermag, dadurch gekennzeichnet, daß der Kuppler der folgenden Formel I entspricht

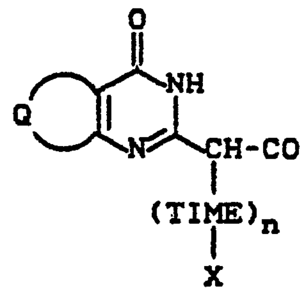

I

worin bedeuten:

$R^1$ Alkyl, eine heterocyclische oder carbocyclische aromatische Gruppe oder $-NH-CO-R^2$;

$R^2$ Alkyl, Aryl oder $-NH-R^3$;

$R^3$ Alkyl oder Aryl;

Q den Rest zur Vervollständigung eines ankondensierten, gegebenenfalls substituierten Benzol- oder heterocyclischen Ringes;

X den Rest einer fotografisch wirksamen Verbindung;

TIME ein Bindeglied, das bei Reaktion des Kupplers mit dem Oxidationsprodukt eines Farbentwicklers zusammen mit dem daran gebundenen Rest X freigesetzt wird und seinerseits unter den Entwicklungsbedingungen den Rest X als fotografisch wirksame Verbindung freisetzt;

n 0 oder 1.

Ein in Formel I durch $R^1$, $R^2$ oder $R^3$ dargestellter Alkylrest ist geradkettig oder verzweigt, substituiert oder unsubstituiert und enthält 1-20 C-Atome; Methyl, Ethyl, Butyl, Hexyl, Dodecyl sind Beispiele hierfür.

Eine in Formel I durch $R^1$ dargestellte aromatische Gruppe kann eine Arylgruppe sein, z.B. Phenyl oder eine heterocyclische Gruppe, z.B. Thiazol, Benzothiazol, Thienyl oder Pyridyl.

Die genannten Gruppen können substituiert sein, z.B. durch Alkyl, Alkoxy, Halogen, Alkoxycarbonyl, Carbamoyl, Sulfamoyl oder Acylamino, wobei der Acylrest von aliphatischen oder aromatischen Carbonsäuren oder Sulfonsäuren oder von Carbaminsäuren oder Kohlensäuremonoestern abgeleitet sein kann. Es ist bevorzugt, wenn $R^1$ Phenyl bedeutet, das mit einem diffusionsfestmachenden Rest, z.B. in Form einer Alkoxy-, Alkoxycarbonyl-, Carbamoyl-, Sulfamoyl-, Sulfonamido- oder Carbonamido- gruppe mit 8 oder mehr Kohlenstoffatomen, substituiert ist. Jedoch kann sich ein diffusionsfestmachender Rest auch als Substituent an dem durch Q vervollständigten Ring befinden oder völlig entfallen. Ein durch Q vervollständigter heterocyclischer Ring ist beispielsweise ein Thiophen-, Pyridin-, Thiazol-, Furan-, Pyrazol-, Benzofuran- oder Thionaphthenring.

Ein in Formel I durch TIME dargestelltes Bindeglied ist eine Gruppe, die nach Abspaltung aus der Kupplungsstelle des Kupplers bei dessen Kupplung mit dem Oxidationsprodukt des Silberhalogenidentwicklungsmittels befähigt ist, in einer Folgereaktion einen daran gebundenen fotografisch wirksamen Rest, im vorliegenden Fall ein Triazol der Formel II freizusetzen. Die Gruppe TIME wird auch als Zeitsteuerglied bezeichnet, wel bei Anwesenheit einer solchen Gruppe der daran gebundene fotografisch wirksame Rest in vielen Fällen verzögert freigesetzt wird und wirksam werden kann. Bekannte Zeitsteuerglieder sind beispielsweise eine Gruppe

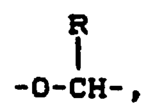

wobei das O-Atom an die Kupplungsstelle des Kupplers und das C-Atom an ein N-Atom einer fotografisch wirksamen Verbindung gebunden ist (z.B. DE-A-27 03 145), eine Gruppe, die nach Abspaltung vom Kuppler einer intramolekularen nucleophilen Verdrängungsreaktion unterliegt und hierbei die fotografisch wirksame Verbindung freisetzt (z.B. DE-A-28 55 697), eine Gruppe, in der nach Abspaltung vom Kuppler eine Elektronenübertragung entlang eines konjugierten Systems stattfinden kann, wodurch die fotografisch wirksame Verbindung freigesetzt wird (z.B. DE-A-31 05 026), oder eine Gruppe

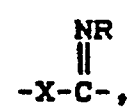

worin X (z.B. -O-) an die Kupplungsstelle des Kupplers und das C-Atom an ein Atom der fotografisch wirksamen Verbindung gebunden ist und worin R beispielsweise für Aryl steht (z.B. EP-A-0 127 063). Die Gruppe TIME kann auch eine Gruppe sein, die nach Abspaltung aus der Kupplungsstelle des Kupplers selbst eine Kupplungsreaktion oder eine Redoxreaktion eingehen kann und als Folge einer solchen Reaktion die an sie gebundene Gruppe X freisetzt.

Bei der durch X dargestellten abspaltbaren Gruppe handelt es sich beispielsweise um ein Halogenatom (z.B. F, Cl, Br, I) oder um eine organische Gruppe, die in der Regel über ein Sauerstoff-, Schwefel- oder Stickstoffatom an die Kupplungsstelle des Kupplermoleküls (gegebenenfalls über eine Gruppe TIME) angeknüpft ist. Falls es sich bei der abspaltbaren Gruppe um eine cyclische Gruppe handelt, kann die Anknüpfung an die Kupplungsstelle des Kupplermoleküls (oder an die Gruppe TIME) entweder direkt über ein Atom, das Bestandteil eines Ringes ist, z.B. ein Stickstoffatom, oder indirekt über ein zwischengeschaltetes Bindeglied erfolgt sein. Derartige abspaltbare Gruppen sind in großer Zahl bekannt, z.B. als Fluchtgruppen von 2-Äquivalentgelbkupplern.

Beispiele von über Sauerstoff angeknüpften abspaltbaren Gruppen entsprechen der Formel

$-O-R^4$,

worin $R^4$ für einen acyclischen oder cyclischen organischen Rest steht, z.B. für Alkyl, Aryl, eine heterocyclische Gruppe oder Acyl, das sich beispielsweise ableitet von einer organischen Carbon- oder Sulfonsäure. Bei besonders bevorzugten abspaltbaren Gruppen dieser Art bedeutet $R^4$ eine gegebenenfalls substituierte Phenylgruppe. Solche Gruppen sind beispielsweise beschrieben in US-A-3 408 194, DE-A-24 56 076.

Beispiele von über Stickstoff angeknüpften abspaltbaren Gruppen sind in den folgenden deutschen Offenlegungsschriften (DE-A-) beschrieben:
20 57 941, 21 63 812, 22 13 461, 22 19 917, 22 61 361, 22 63 875, 23 18 807, 23 29 587, 23 44 155, 23 63 675, 24 33 812, 24 41 779, 24 42 703, 25 28 638, 25 28 860, 26 37 817, 28 18 373, 30 20 416.

Hierbei handelt es sich durchweg um 5- oder 6-gliedrige heterocyclische Ringe, die über ein Ringstickstoffatom mit der Kupplungsstelle des Kupplers verbunden sind. Die heterocyclischen Ringe enthalten vielfach benachbart zu dem die Bindung an das Kupplermolekül vermittelnden Stickstoffatom aktivierende Gruppen, z.B. Carbonyl- oder Sulfonylgruppen oder Doppelbindungen.

Wenn die abspaltbare Gruppe über ein Schwefelatom an die Kupplungsstelle des Kupplers gebunden ist, kann es sich bei ihr um den Rest einer diffusionsfähigen Mercaptoverbindung handeln, die die Entwicklung von Silberhalogenid zu inhibieren vermag. Derartige Inhibitorreste sind vielfach als an die Kupplungsstelle von Kupplern, auch offenkettigen Ketomethylenkupplern gebundene abspaltbare Gruppe beschrieben werden, z.B. in US-A-3 227 554.

Bei der abspaltbaren Gruppe X handelt es sich bevorzugt um den Rest einer fotografisch wirksamen Verbindung, die über ein Stickstoffatom eines 1,2,3- oder 1,2,4-Triazolringes an die Kupplungsstelle des Kupplers oder an das Zeitsteuerglied TIME gebunden ist. Eine solche Gruppe X entspricht beispielsweise der Formel

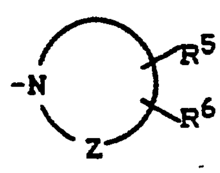

worin bedeuten:
Z den Rest zur Vervollständigung eines 1,2,3- oder 1,2,4-Triazolringes;
$R^5$ und $R^6$ H, Alkyl, Aryl, eine heterocyclische Gruppe, Alkoxy, $-S-R^7$, Amino, Acylamino, eine Carbonsäureestergrupe oder $-CO-NR^8R^9$,
oder $R^5$ und $R^6$ bedeuten zusammen (falls der durch 2 vervollständigte Ring ein 1,2,3-Triazolring ist) den Rest zur Vervollständigung eines ankondensierten, gegebenenfalls substituierten vorzugsweise aromatischen Ringes, z.B. eines Tetrahydrobenzolringes, eines Benzolringes oder eines Thiophenringes,
wobei gilt, daß wenigstens einer der Reste $R^5$ und $R^6$ eine Gruppe mit fotografischer Wirksamkeit darstellt oder daß die Verbindung der Formel II

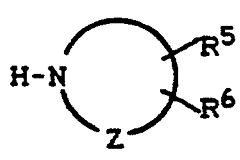

II

nach Freisetzung als Ganzes eine fotografisch wirksame Verbindung ist;
$R^7$ Alkyl, Cycloalkyl, Aralkyl, Alkenyl, Alkinyl oder Aryl;
$R^8$ Alkyl, Aralkyl oder Aryl;
$R^9$ H, einen Rest wie $R^8$
oder $R^8$ und $R^9$ zusammen den Rest zur Vervollständigung einer cyclischen Aminogruppe.

Ein in Formel II durch $R^5$, $R^6$, $R^7$, $R^8$ oder $R^9$ dargestellter Alkylrest kann geradkettig oder verzeigt sein und bis zu 10 C-Atomen enthalten; Beispiele sind Methyl, Ethyl, Propyl, Isopropyl, Butyl, s-Butyl, t-Butyl, Pentyl, Hexyl, Octyl. Die Alkylreste können substituiert sein, z.B. durch Hydroxyl, Alkoxy, Alkylthio, Acylamino oder eine cyclische Imidgruppe.

Ein durch $R^7$ dargestellter Cycloalkylrest ist beispielsweise Cyclohexyl; ein Aralkylrest ($R^7$, $R^8$) ist beispielsweise Benzyl; ein Alkenylrest ist beispielsweise Allyl oder 2-Butenyl; en Alkinylrest ist beispielsweise Propinyl.

Eine cyclische Aminogruppe ($R^8$, $R^9$) ist beispielsweise eine Piperidino- oder Morpholinogruppe.

Eine cyclische Imidogruppe ist beispielsweise eine Succinimidogruppe, Maleinimidogruppe, Phthalimidogruppe, Hexahydrophthalimidogruppe oder eine Gruppe der Formel

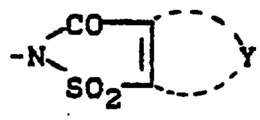

worin
Y den zur Vervollständigung eines carbocyclischen oder heterocyclischen, gegebenenfalls substituierten Ringes erforderlichen Rest bedeutet.

Eine durch $R^5$ oder $R^6$ dargestellte heterocyclische Gruppe ist beispielsweise eine Furyl-, Thiazolyl- oder 1,2,4-Triazolylgruppe. Eine solche heterocyclische Gruppe kann weitere Substituenten tragen, z.B. Alkyl, Alkoxy, Alkylthio (-S-$R^7$).

Die vorteilhaften Eigenschaften der erfindungsgemäßen Kuppler sind vermutlich darauf zurückzuführen, daß der Triazolring offenbar nicht nur eine gute Abgangsgruppe (Fluchtgruppe) ist, so daß die Kuppler sehr reaktiv sind, sondern offenbar auch eine gewisse Neigung hat sich am Silberhalogenidkorn zu adsorbieren und hierbei die sich bei der Entwicklung des Silberhalogenids abspielenden Vorgänge zu beeinflussen. Die die Wirksamkeit der fotografisch wirksamen Verbindung bestimmenden Gruppen kommen hierbei offenbar in besonders günstigen Kontakt mit der Oberfläche des Silberhalogenidkorns. Es ist daher erfindungsgemäß bevorzugt, wenn die fotografisch wirksame Verbindung eine solche ist, die die Entwicklung des Silberhalogenids beeinflußt, z.B. ein Entwicklungsbeschleuniger, ein Verschleierungsmittel, ein Bleichbeschleuniger oder besonders bevorzugt ein Entwicklungsinhibitor. Namentlich im letzteren Fall ist es dabei weiter bevorzugt, wenn von den Resten $R^5$ und $R^6$ mindestens einer für -S-$R^7$, Alkoxycarbonyl mit 2 bis 10 C-Atomen oder eine heterocyclische Gruppe steht.

Die erfindungsgemäßen Kuppler liefern bei der chromogenen Entwicklung durch Kupplung mit Farbentwickleroxidationsprodukten gelbe bis gelbrötliche Farbstoffe. Ihre Hauptbedeutung liegt darin, daß sie bei der Entwicklung bildmäßig die jeweilige fotografisch wirksame Verbindung freisetzen. Da sie sehr aktiv sind, können sie in vergleichsweise geringen Konzentrationen verwendet werden.

Dies erleichtert ihren Einsatz auch in solchen Schichten des farbfotografischen Aufzeichnungsmaterials, in denen hauptsächlich Purpur- oder Blaugrünfarbstoffe erzeugt werden, ohne daß das jeweilige Farbbild durch eine gelbe Nebendichte wesentlich beeinträchtigt wird.

Im Sinne der Erfindung besonders brauchbare DIR-Kuppler sind beispielsweise folgende:

D-1

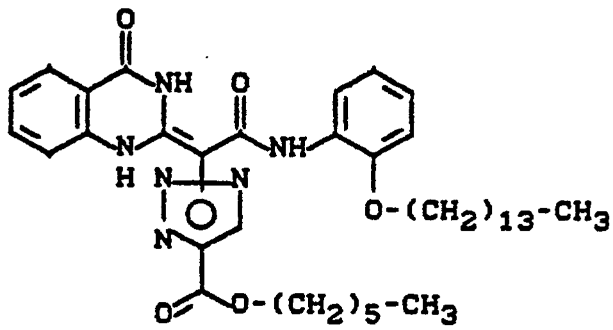

D-2

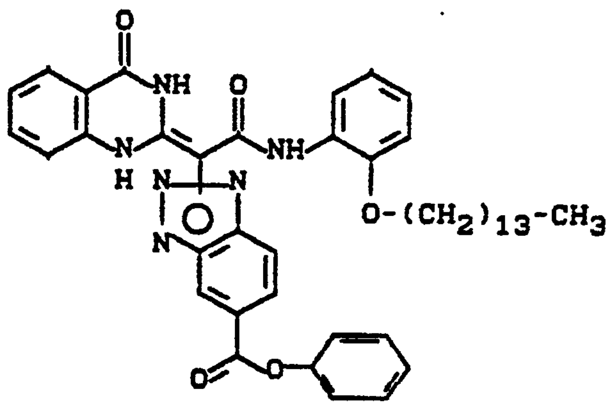

D-3

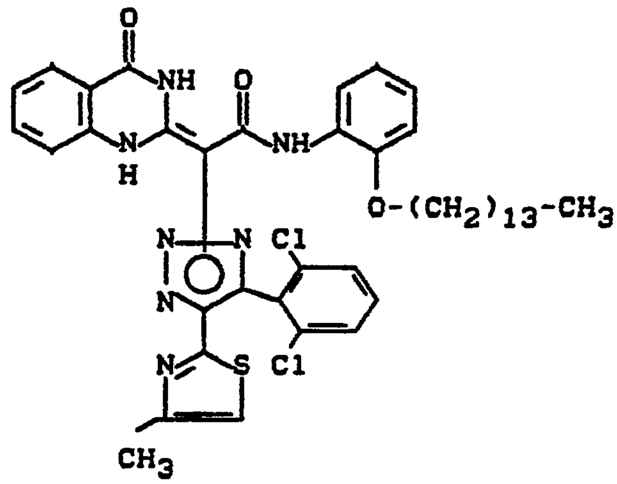

D-4

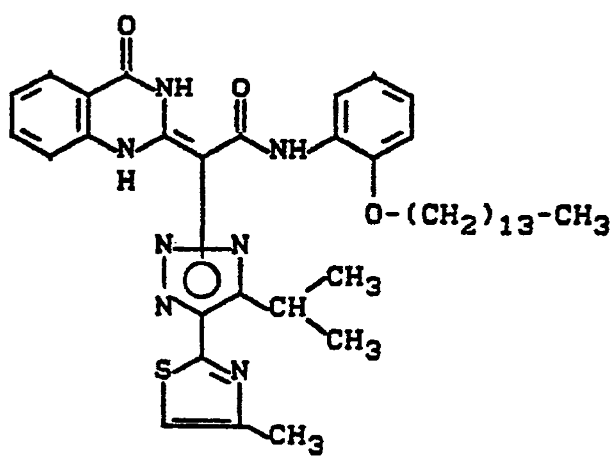

D-5

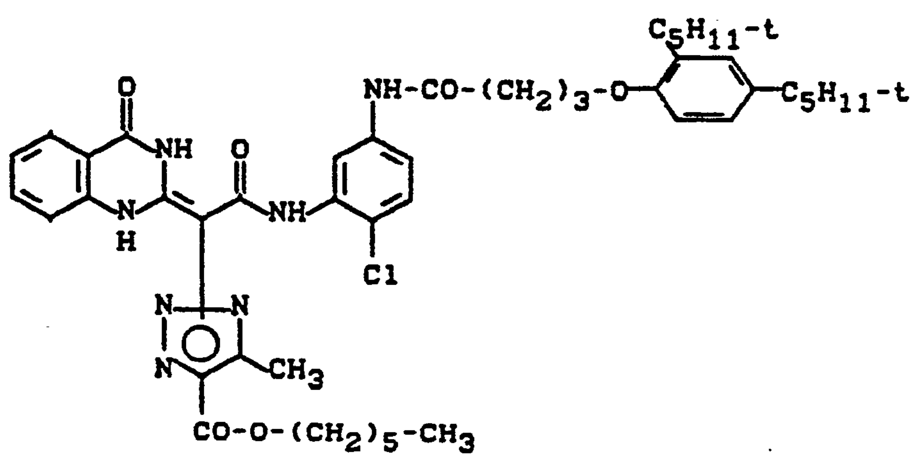

D-6

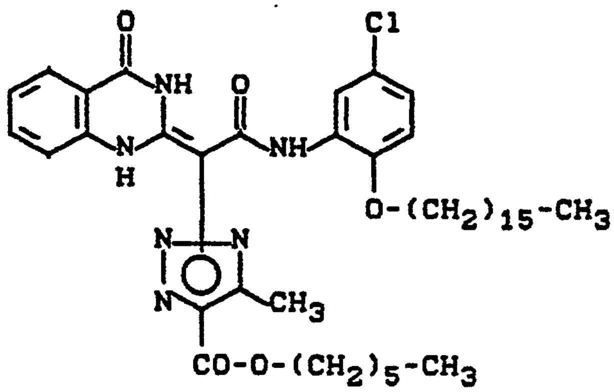

D-7

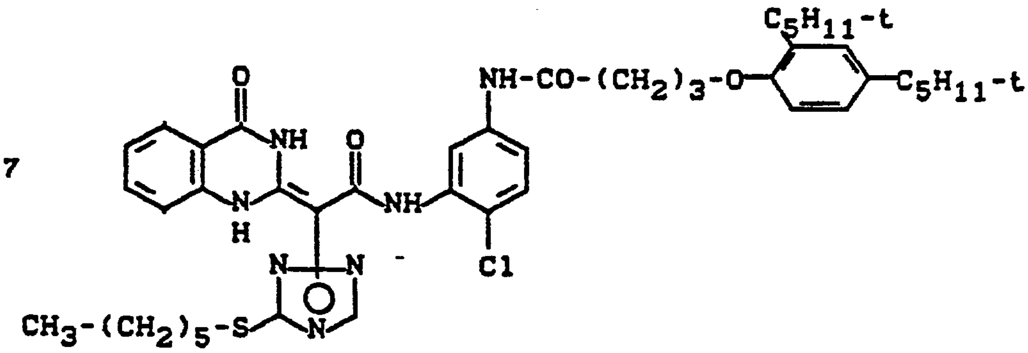

D-8

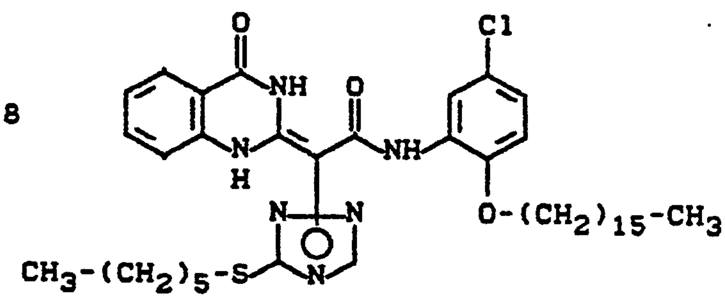

D-9

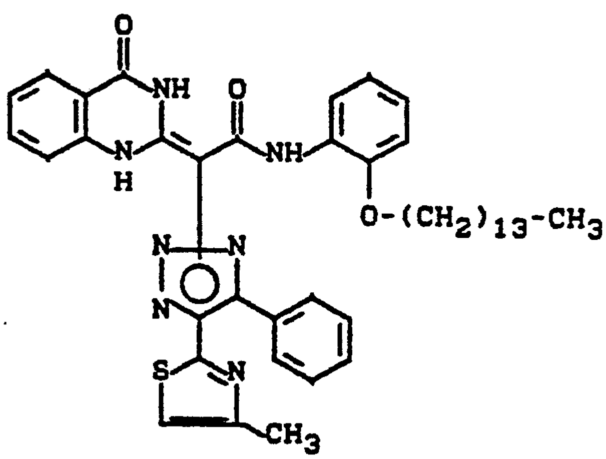

D-10

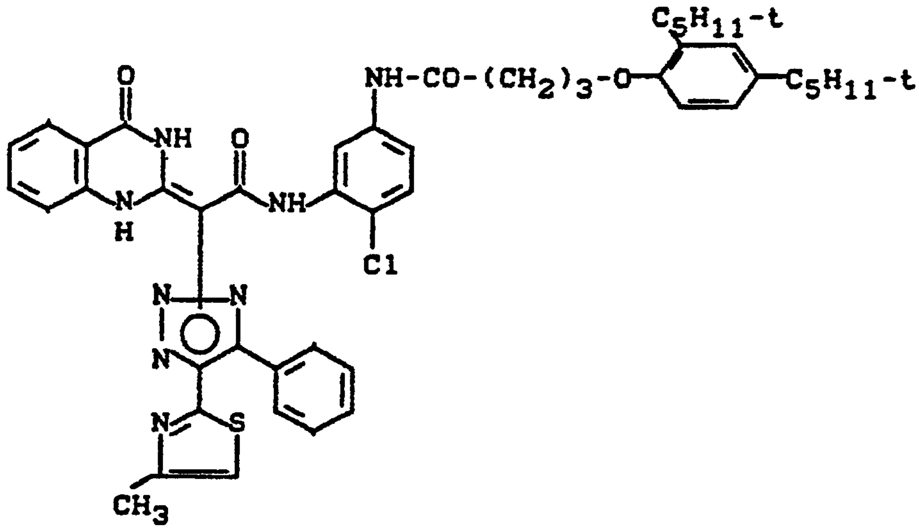

D-11

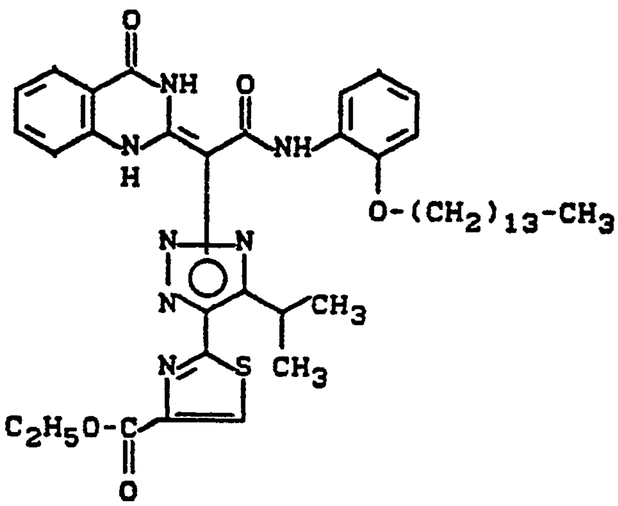

D-12

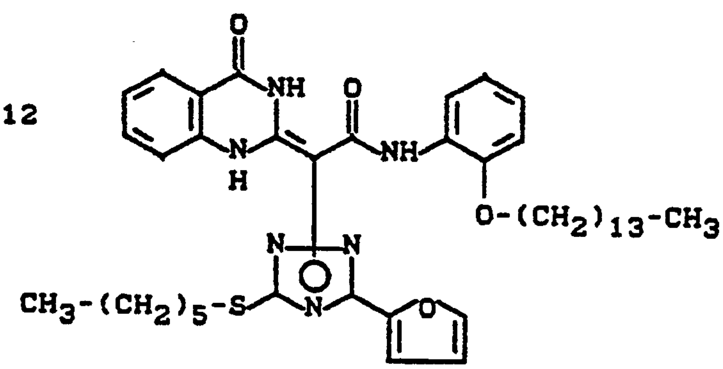

D-13

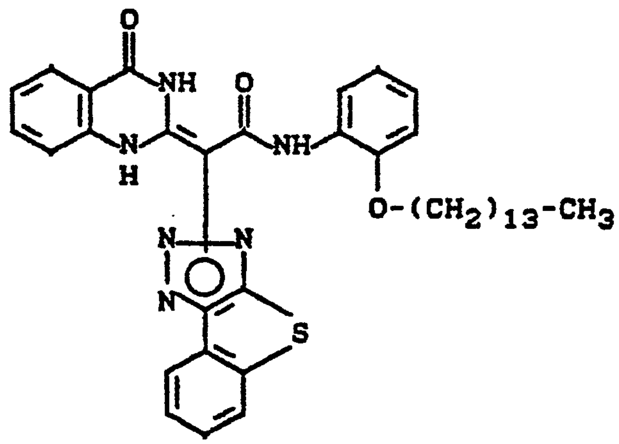

D-14

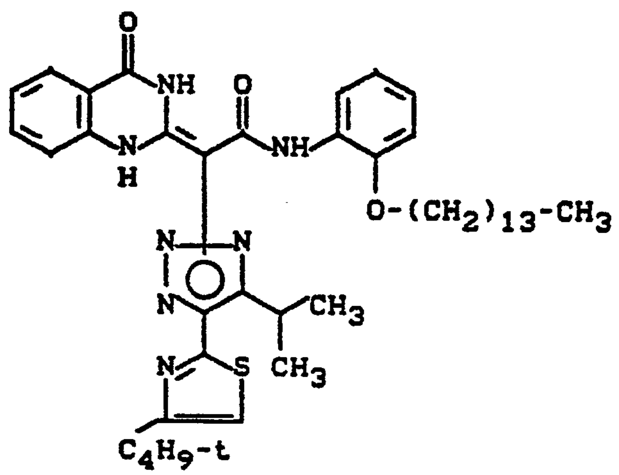

D-15

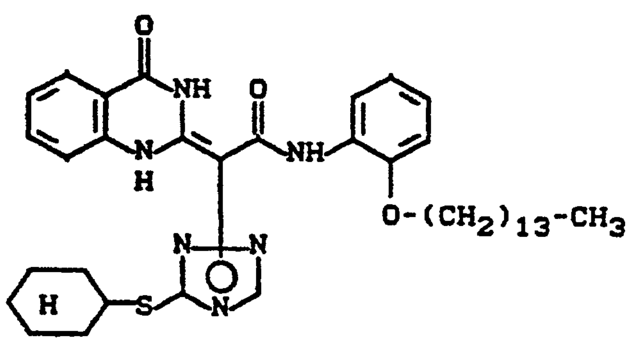

D-16

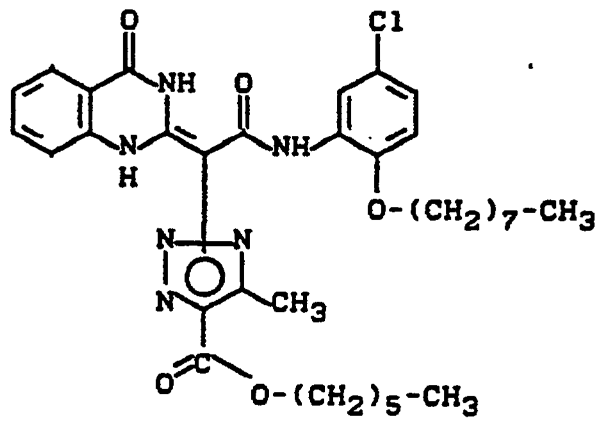

D-17

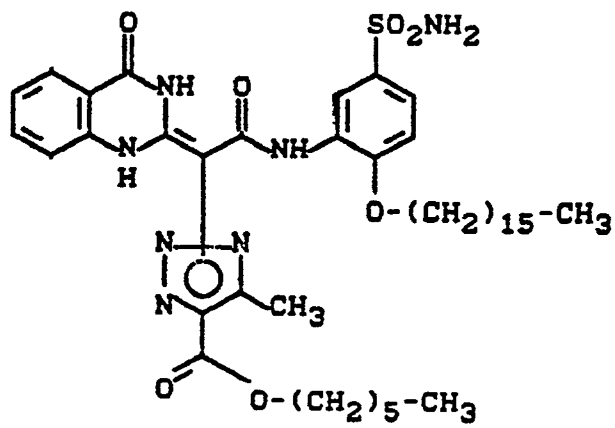

D-18

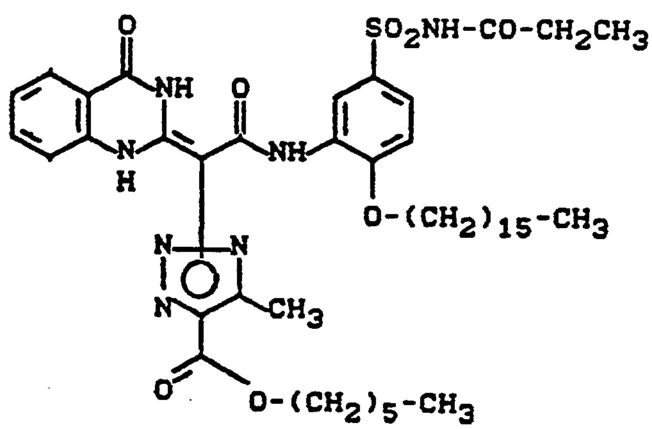

D-19

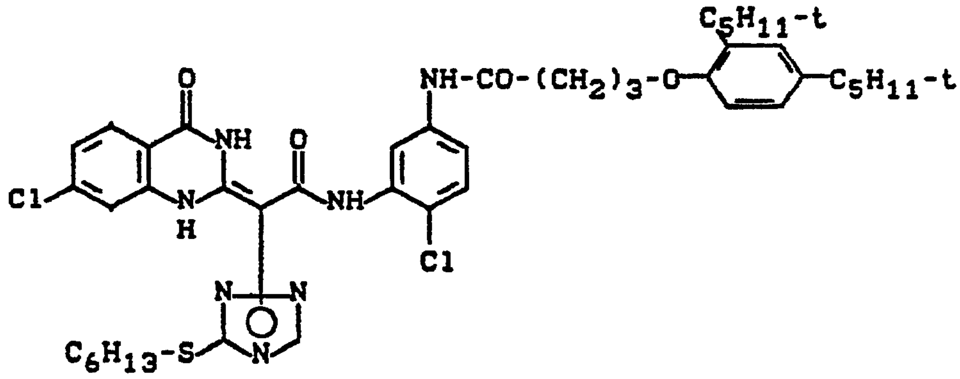

D-20

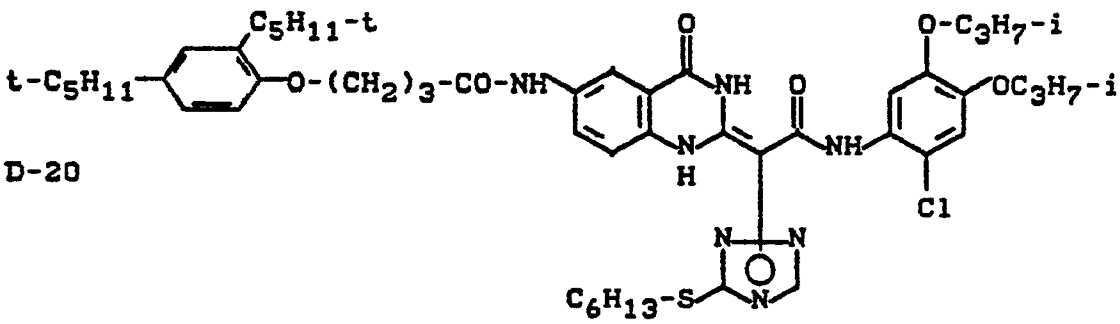

D-21

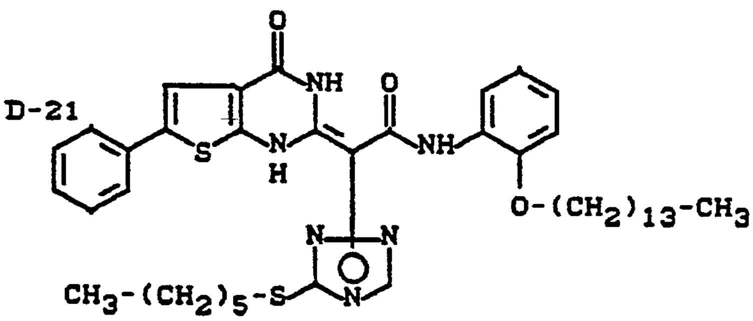

D-22

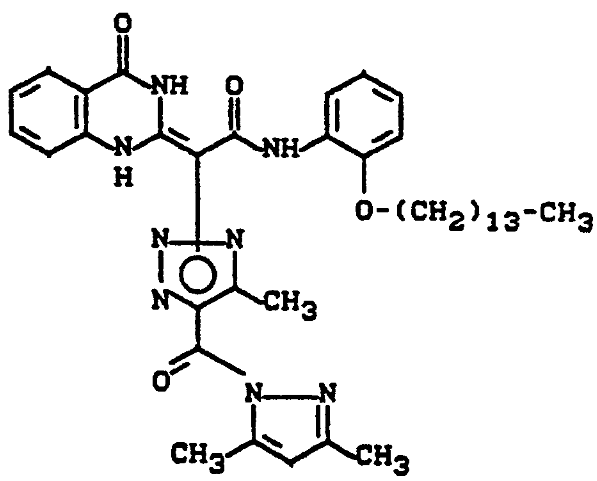

Die Herstellung der erfindungsgemäßen Kuppler kann in der Weise erfolgen, daß zunächst die Ester der allgemeinen Formel III hergestellt werden

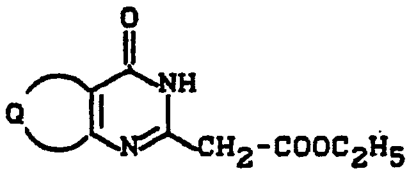

III

diese dann in bekannter Weise in die entsprechenden 4-Äquivalentkuppler der allgemeinen Formel IV umgewandelt werden

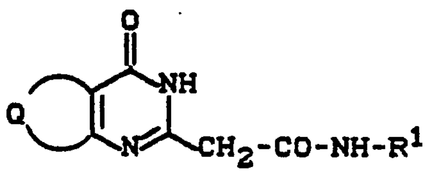

IV

worauf aus letzteren dann durch Einführung der Gruppe

$-(TIME)_n-X$

in die Kupplungsstelle die erfindungsgemäßen Kuppler erhalten werden.

Zur Herstellung der Anthranilamide (Ausgangsmaterial zur Herstellung der Chinazolon-2-essigester (III)) dienen durchweg bekannte Verfahren:

1) Ammonolyse von Isatosäureanhydriden
2) Aminolyse von Anthranilsäureestern
3) partielle Verseifung von 2-Aminobenzonitrilen mit Säure oder Alkali
4) direkte Ringsynthesen

Geeignete Vorstufen sind beispielsweise die folgenden:

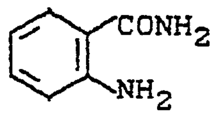

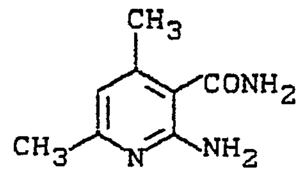

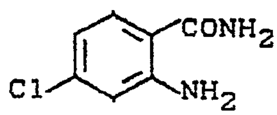

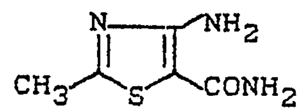

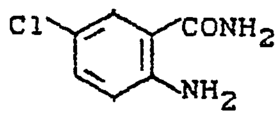

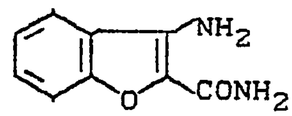

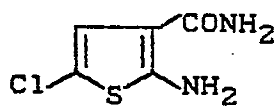

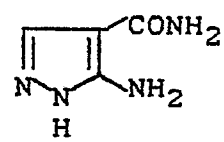

Geeignete Ester gemäß Formel III sind beispielsweise die folgenden:

Chinazolon-2-essigester:

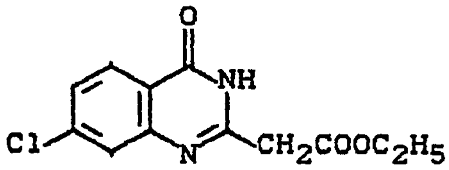

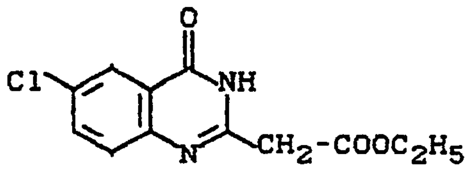

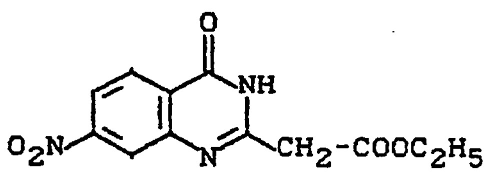

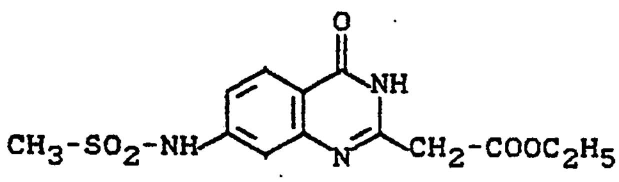

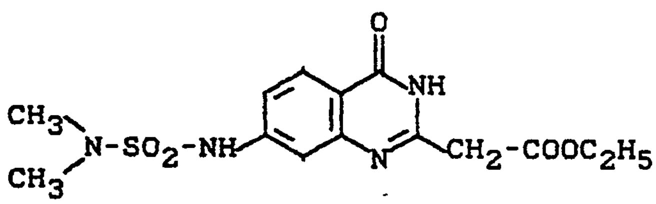

Thienopyrimidin-4-on-2-essigester:

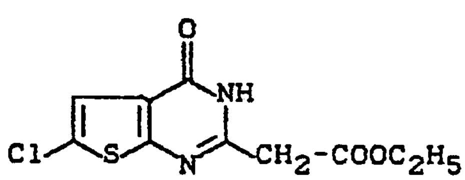

Verfahren zu ihrer Herstellung (z.B. Umsetzung von Maloniminoether-ester mit 2-Aminoaryl-carbonamiden oder Umsetzung von β,β-Dialkoxy-acrylsäureestern mit 2-Aminocarbonamiden) sind beispielsweise in GB-A-689 023 beschrieben.

Die Ester III können auch wie folgt hergestellt werden:

- Umsetzung von halogenierten Chinazolonen mit Acetessigester
- Schwefelextrusion aus Chinazolon-2-thioglykolsäureestern mit starker Base oder mit thiophilen Reagentien (z.B. Triphenylphosphin)

Die Umsetzung der Ester III zu den Amiden IV kann entweder direkt durch Aminlyse, vorzugsweise bei höheren Temperaturen >120°C, erfolgen oder auf dem Umweg über die freie Säure, die aus dem Ester durch vorsichtige Verseifung mit Alkali zugänglich ist.

Besonders geeignete Vieräquivalentkuppler IV, aus denen die entsprechenden Zweiäquivalentkuppler gemäß vorliegender Erfindung hergestellt werden können, sind beispielsweise folgende:

IV-1

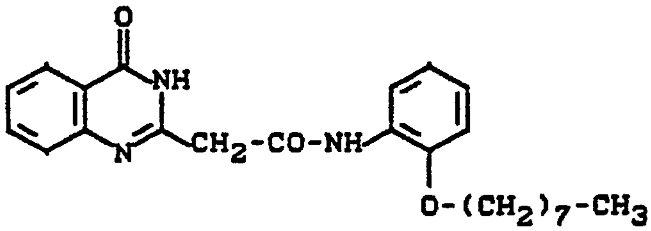

IV-2

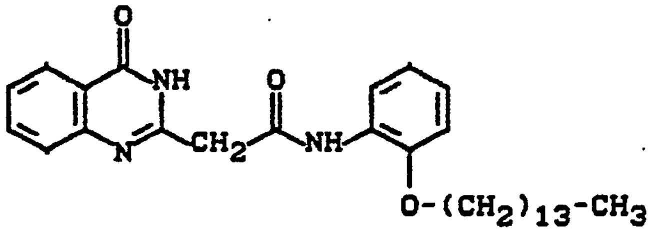

IV-3

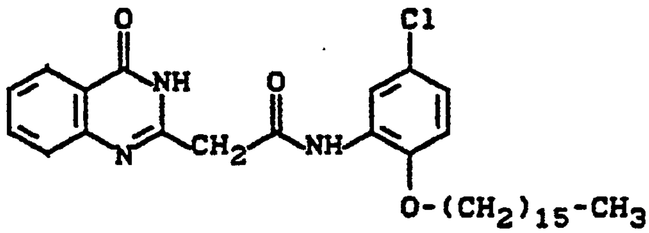

IV-4

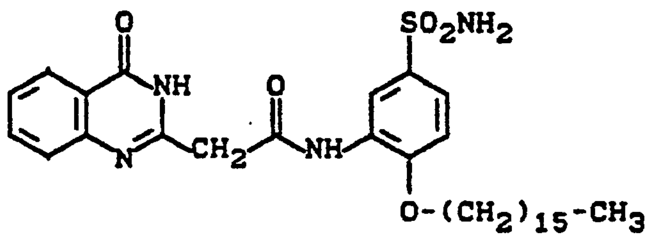

IV-5

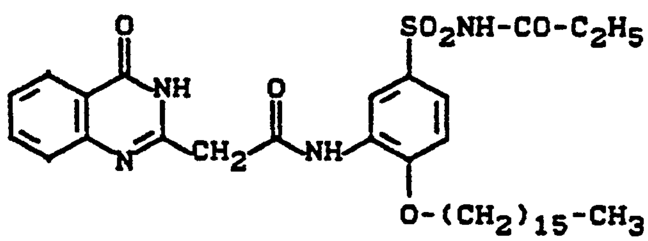

IV-6

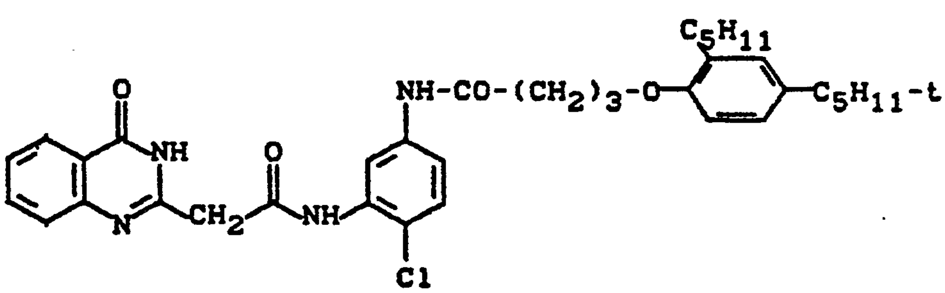

IV-7

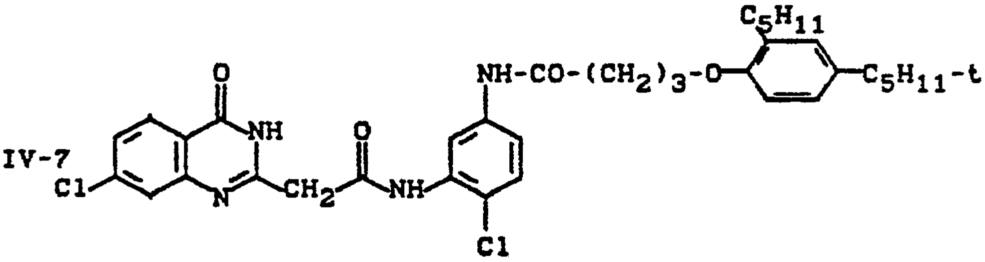

IV-8

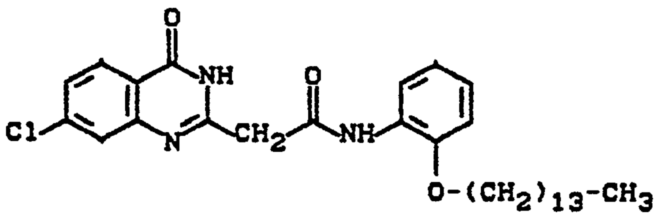

IV-9

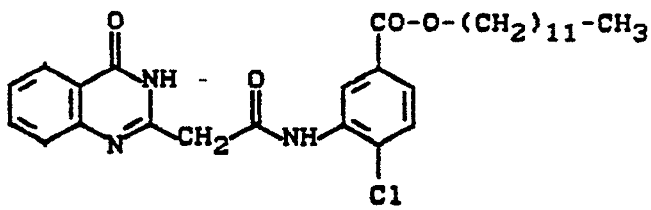

IV-10

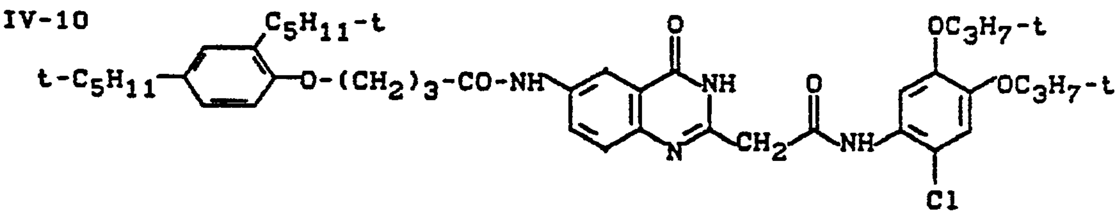

IV-11

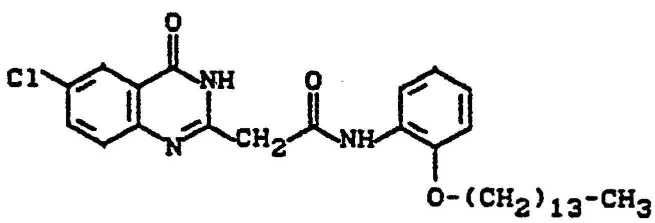

Aus den Chinazolonacetamiden IV sind die erfindungsgemäßen Zweiäquivalentkuppler durch bekannte Verfahren der Zweiäquivalent-Kupplerchemie zugänglich, beispielsweise durch Umsetzung der halogenierten Kuppler der allgemeinen Formel V (z.B. erhalten aus den 4-Äquivalentkupplern IV durch einfache Halogenierung mit Brom, Chlor oder Sulfurylchlorid) mit einem Triazol II.

V 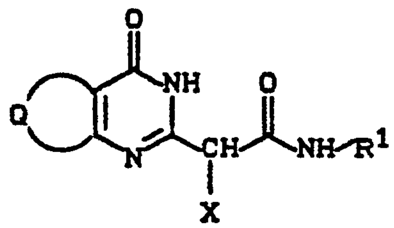 X = Cl, Br

oder

VI 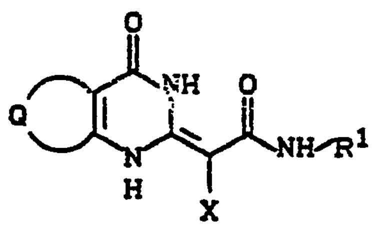

Nach den Ergebnissen der Protonenresonanzspektren liegen alle untersuchten halogenierten Chinazolon-2-acetamide in der Ketenaminalform VI vor, doch ist nicht auszuschließen, daß einzelne Verbindungen im tautomeren Gleichgewicht mit der Form V vorliegen.

Besonders geeignete halogenierte Kuppler V, z.B. aufgrund ihrer Reaktivitäts-, Löslichkeits- oder Stabilitätseigenschaften sind die folgenden:

V-2

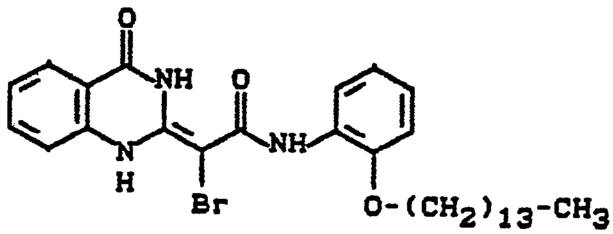

V-3

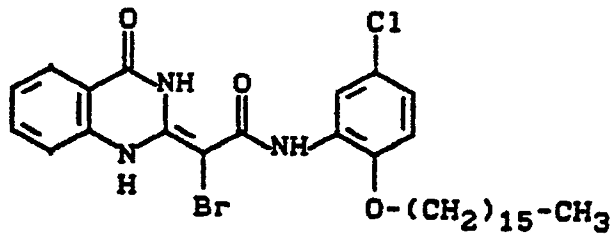

V-4

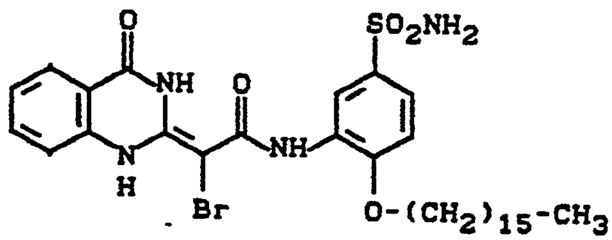

V-6

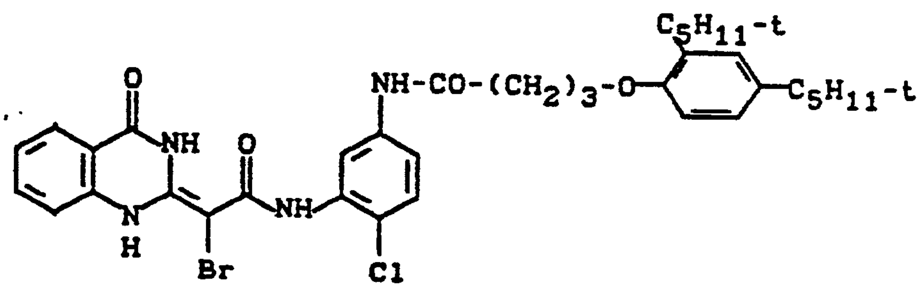

V-11

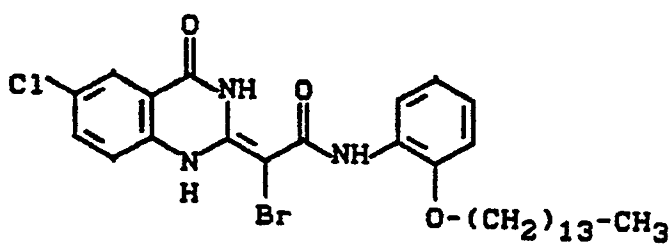

Die Verknüpfungsstelle des Triazolringes mit dem Chinazolonmethinrest ist im einzelnen nicht bekannt; wegen der ausgeprägten Delokalisation der Ladung wird auch meistens bei der Verknüpfung keine Stelle besonders bevorzugt. Im allgemeinen fallen bei der Synthese der Verbindungen Isomerengemische an, die wegen der gleichartigen Reaktivität keineswegs aufgetrennt zu werden brauchen.

Beispiele für geeignete Triazole (erfindungsemäß bevorzugte freisetzbare Gruppen X meist mit Entwicklungsinhibitorwirkung) sind im folgenden aufgeführt:

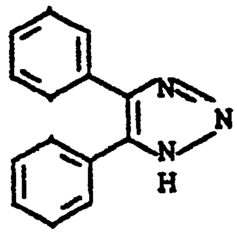
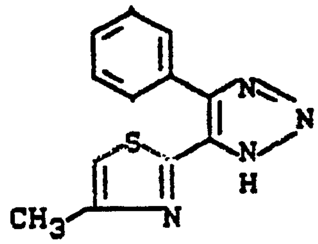
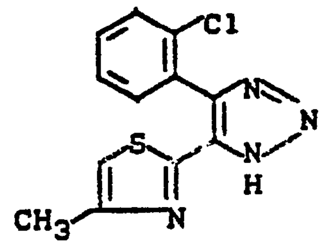

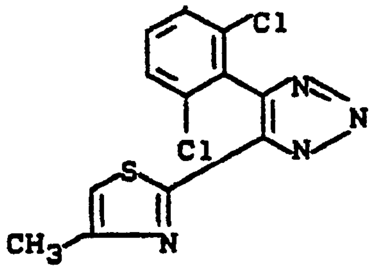
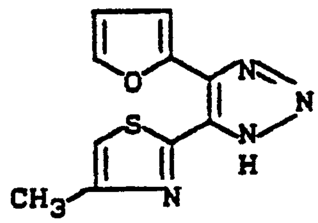

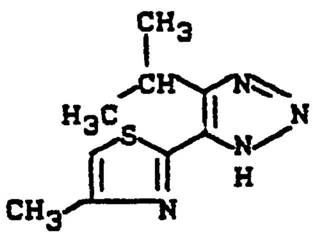
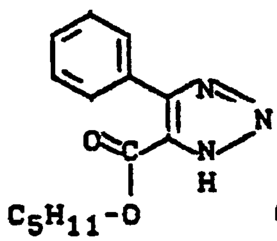
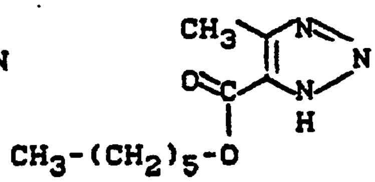

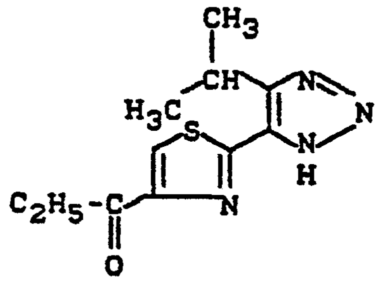

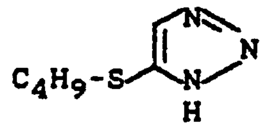

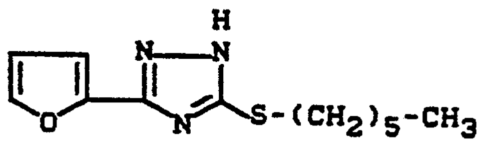

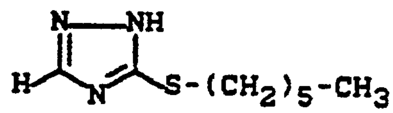

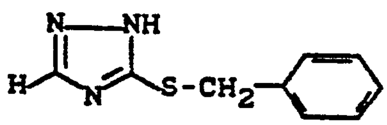

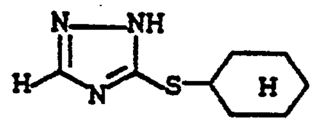

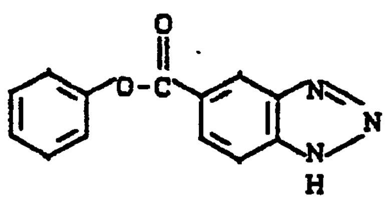

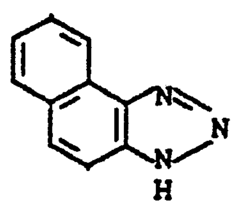

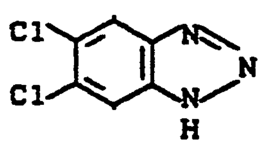

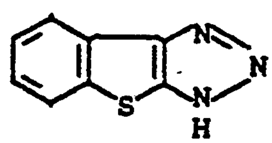

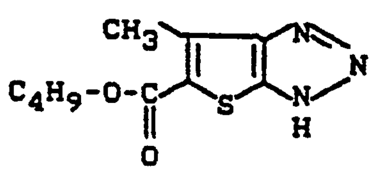

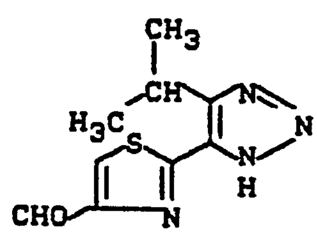

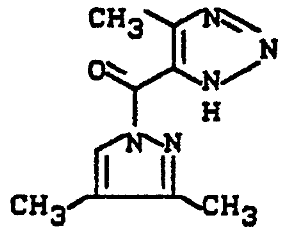

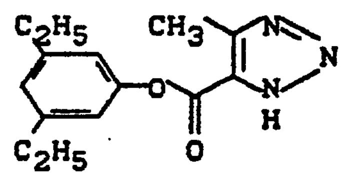

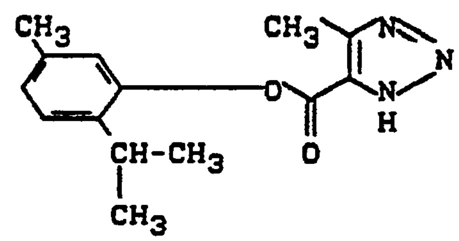

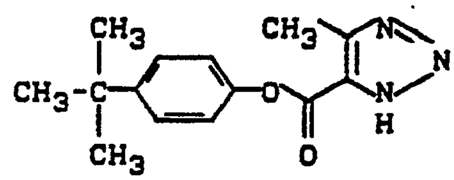

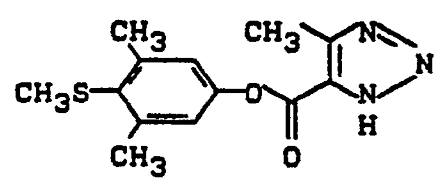

Entwicklungsinhibierende, entwicklungsbeschleunigende oder keimbildende Wirkung kann auch Triazolverbindungen zukommen, die erst nach der Abspaltung vom Kupplerrest durch einen nachfolgenden Hydrolyseschritt in die eigentliche wirksame Triazolverbindung übergehen, z.B. unter Abspaltung einer Acylfunktion oder Aufspaltung eines Lactonringes. Schließlich kann auch die eigentlich wirksame entwicklungsbeschleunigende oder nukleierend wirkende Verbindung durch einen Hydrolyseschritt oder einen Eliminierungsschritt, bzw. eine intramolekulare nukleophile Verdrängungsreaktion, bei der ein unter Umständen nur kurzlebiger Ring gebildet wird, aus der im Kupplungsschritt freigesetzten Triazolverbindung abgespalten werden.

Einen Sonderfall stellen Triazole dar, die einen Rest enthalten, der selbst kupplungsfähig ist oder durch einen Hydrolyseschritt in einen kupplungsfähigen Rest übergeht.

Als DAR-(Development Accelerator Releasing)Kuppler wirken demnach erfindungsgemäße Zweiäquivalentkuppler, die eine Triazolverbindung mit entwicklungsbeschleunigender Wirkung oder mit keimbildender Wirkung abspalten. Diese Wirkung kann auf verschiedene Weise zustandekommen, z.B. durch die Fähigkeit der abgespaltenen Triazolverbindung, mit $Ag^{+}$-Ionen lösliche Komplexe zu bilden, durch Verbrauch von Farbentwickleroxidationsprodukt in höherer Geschwindigkeit als der ursprüngliche Kuppler, durch Ausbildung eines superadditiven Systems in Kombination mit dem Farbentwickler und schließlich durch reduktive Bildung von entwickelbaren Silberkeimen auf vorher nicht entwickelbaren Silberhalogenidkörnern in der Umgebung des entwickelnden Silverhalogenidkristalls. Dementsprechend können die in den erfindungsgemäßen Kupplern I vorliegenden Triazolverbindungen Substituenten enthalten, die die genannten Effekte ermöglichen, zum Beispiel Substituenten mit einer Bisthioetherstruktur, einer Hydrazidstruktur, einer Phenylhydrazidstruktur, einer Thioetherhydrazidstruktur, einer Thioharnstoff-, Dithiocarbamat- oder Rhodaninstruktur.

## Herstellungsbeispiele

### Kuppler IV-2

Man erhitzt eine Lösung von 34,8 g (0,15 mol) 4-Keto-3,4-dihydrochinazolin-2-essigsäureethylester (hergestellt nach Beispiel 1 aus GB-A-689 023) und 45,8 g 2-Tetradecyloxyanilin in 150 ml p-Cymol unter Abdestillieren des freigesetzten Ethanols 3 h unter Rückfluß, rührt in 400 ml Methanol ein, läßt über Nacht auskristallisieren, saugt ab und wäscht mit Methanol nach. Nach Kristallisation aus Essigester oder Acetonitril erhält man 49 g weiße Kristalle mit dem Schmelzpunkt 117 - 120°C.

In gleicher Weise werden folgende 4-Äquivalentkuppler hergestellt:

IV-1 Fp. 152-154°C
IV-3 Fp. 135-137°C
IV-4 Fp. >140°C unter allmählicher Zersetzung
IV-6 Fp. 166-170°C
IV-7 Fp. 208-210°C (aus Essigsäure)
IV-8 Fp. 142-143°C
Alle Schmelzpunkte sind unkorrigiert.

Verbindung V-2 (bromierter Kuppler)

Zu einer Suspension von 29,5 g (0,06 mol) Kuppler IV-2 in 300 ml Essigsäure tropft man unter Rühren bei 25°C eine Lösung von 9,6 (0,06 mol) Brom in 50 ml Essigsäure. Nach beendeter Bromierung werden 40 ml Wasser zugegeben. Man läßt über Nacht stehen, verdünnt mit 400 ml Wasser und saugt ab. Man wäscht mit Methanol nach und trocknet an der Luft. Man erhält 27,4 g schwach cremefarbiges Pulver mit dem Zersetzungsschmelzpunkt 130-132°C. Zur Reinigung kristallisiert man aus Methylethylketon um. Nach dem Ergebnis der Dünnschichtchromatographie (Kieselgel, Laufmittel Toluol-Ethylacetat 7:3) enthält das gereinigte Produkt keine Dibromverbindung.

Nach demselben Verfahren werden z.B. folgende Verbindungen erhalten:

V-3 Fp. 135-137°C
V-6 Fp. 181-183°C

Kuppler D-5 (erfindungsgemäßer Kuppler)

Zu einer Suspension von 14,1 g Verbindung V-6 (0,02 mol) und 4,6 g 4-Methyl-1,2,3-triazol-5-carbonsäure-n-hexylester (0,024 mol) in 50 ml Dimethylacetamid tropft man bei Raumtemperatur 2,8 g 1,1,3,3-Tetramethylguanidin. Die gelbrote Lösung wird nach 1 h in 200 ml Eiswasser gegossen. Der flockige Niederschlag wird abgesaugt, durch Chromatographie mit Toluol-Ethylacetat auf Kieselgel von Verunreinigungen befreit und aus Acetonitril umkristallisiert. Man erhält 6,7 g blaßgelbe Kristalle mit dem Schmelzpunkt 128°C.

Nach dem gleichen Verfahren erhält man folgende Kuppler, zumeist in Form von Isomerengemischen.

Verbindung:

D-1 Fp. 115-120°C
D-2 erweicht oberhalb 170°C, Fp. 195-200°C
D-3 Fp. 131-133°C
D-4 2 Isomere nach präparativer Trennung
Isomer A: Fp. 112-114°C
Isomer B: Fp. 137-139°C

Obwohl theoretisch die Bildung von 3 Isomeren möglich ist, werden in den meisten Fällen im Temperaturbereich 10 - 55°C nur 2 Isomere gebildet, die Bildung eines dritten, gelegentlich intensiv fluoreszierenden Isomeren wird erst bei Umsetzungstemperaturen oberhalb 75°C in nennenswertem Umfang beobachtet. Dieses Isomer wird bei der fraktionierten Kristallisation des Rohproduktes meist über die Mutterlaugen entfernt.

Die einzelnen Isomeren weisen bei den meisten Zweiäquivalentkupplern vergleichbare Kupplungsaktivitäten auf, obwohl sie sich in der Löslichkeit z.T. beträchtlich unterscheiden. Die Isomerenverteilung ist daher nicht kritisch und zumeist durch sterische Gründe vorgegeben. Sie kann aber auch durch die Reaktionsparameter beeinflußt werden, insbesondere durch

1. die Polarität des Lösungsmitels,
2. den Wassergehalt des Reaktionsmediums,
3. die Wahl der Hilfsbase,
4. die Umsetzungstemperatur.

Im allgemeinen wird ein wasserfreies Medium bevorzugt, weil die Umsetzung darin sauberer und einheitlicher, wenn auch nicht unbedingt schneller abläuft.

Als Lösungsmittel kommen für die Umsetzung zwischen halogeniertem Kuppler und Triazolverbindung in erster Linie folgende in Frage:
Diemthylformamid, Dimethylacetamid, N-Methylpyrrolidon, Tetramethylharnstoff, Sulfolan, Ethylacetat, Butylacetat, Acetonitril, Methylethylketon, Aceton, Cyclohexanon, Chlorbenzol, Toluol, Diethylcarbonat.

An Hilfsbase wird im allgemeinen nicht mehr als 1 Äquivalent benötigt. Überschüsse schaden jedoch nicht und komplizieren auch die Aufarbeitung nicht. Als Hilfsbasen eignen sich organische oder anorganische Basen, wobei hohe Basizität keine besonderen Vorteile bietet. Besonders günstig sind: Tetramethylguanidin, Diazabicycloundecan, Diazabicyclo-nonan, Natriumcarbonat, Kaliumcarbonat, Kalium-tert.-butylat.

Auch die Reihenfolge der Zugabe der einzelnen Komponenten ist nicht kritisch, zumal die halogenierten Kuppler im allgemeinen schwerlöslich sind und erst im Lauf der Reaktion in Lösung gehen.

Überraschend ist der Löslichkeitsanstieg, der mit der Umsetzung zum triazolsubstituierten Zweiäquivalentkuppler im allgemeinen verbunden ist und deren problemlose Verwendung in einem fotografischen Aufzeichnungsmaterial erleichtert. Es ist anzunehmen, daß diese Löslichkeits verbesserung mit der Ausbildung von intramolekularen Wasserstoffbrückenbindungen zusammenhängt, die dem Molekül die Ausbildung von Isomeren und Tautomeren in großer Zahl ermöglicht.

Die Verbindungen der vorliegenden Erfindung eignen sich beispielsweise für die Verwendung als Gelb-DIR-Kuppler in farbfotografischen, insbesondere mehrschichtigen Aufzeichnungsmaterialien. Als Gelbkuppler werden sie bevorzugt in oder zugeordnet zu einer lichtempfindlichen Silberhalogenidemulsionsschicht mit einer überwiegenden Empfindlichkeit für den blauen Spektralbereich des sichtbaren Lichtes verwendet. Der besondere Vorteil der erfindungsgemäßen Gelb-DIR-Kuppler, nämlich eine vergleichsweise geringe Entwicklungsinhibierung in der Schicht, der eine solche Verbindung zugeordnet ist, neben einer vergleichsweise hoben Entwicklungsinhibierung in benachbarten nicht zugeordneten Schichten, kommt naturgemäß besonders dann zum Tragen, wenn es sich um ein mehrschichtiges farbfotografisches Aufzeichnungsmaterial handelt, das neben einer überwiegend blauempfindlichen Silberhalogenidemulsionsschicht weitere lichtempfindliche Silberhalogenidemulsionsschichten enthält mit überwiegender Empfindlichkeit für den grünen bzw. roten Spektralbereich des sichtbaren Lichtes.

Die erfindungsgemäßen DIR-Kuppler können wegen ihrer außerordentlich hohen Wirksamkeit in vergleichsweise geringen Mengen eingestzt werden um die erwünschten Effekte, insbesondere die Interimageeffekte hervorzubringen. Dies ermöglicht es, die erfindungsgemäßen DIR- Kuppler nicht nur in den blauempfindlichen Gelbfarbstoff erzeugenden Schichten sondern auch in anderen Schichten einzusetzen, ohne daß dort eine zu hohe unerwünschte Nebendichte auftritt. Die erfindungsgemäßen DIR-Kuppler sind somit mit Vorteil auch in Purpurschichten wie auch in Blaugrünschichten anwendbar.

Bei der Herstellung des lichtempfindlichen farbfotografischen Aufzeichnungsmaterials können die diffusionsfesten DIR-Kuppler der vorliegenden Erfindung gegebenenfalls zusammen mit anderen Kupplern in bekannter Weise in die Gießlösung der Silberhalogenidemulsionsschichten oder anderer Kolloidschichten eingearbeitet werden. Beispielsweise können öllösliche oder hydrophobe Kuppler vorzugsweise aus einer Lösung in einem geeigneten Kupplerlösungsmittel (Ölbildner) gegebenenfalls in Anwesenheit eines Netz- oder Dispergier mittels zu einer hydrophilen Kolloidlösung zugefügt werden. Die hydrophile Gießlösung kann selbstverständlich neben dem Bindemittel andere übliche Zusätze enthalten. Die Lösung des Kupplers braucht nicht direkt in die Gießlösung für die Silberhalogenidemulsionsschicht oder eine andere wasserdurchlässige Schicht dispergiert zu werden; sie kann vielmehr auch vorteilhaft zuerst in einer wäßrigen nichtlichtempfindlichen Lösung eines hydrophilen Kolloids dispergiert werden, worauf das erhaltene Gemisch gegebenenfalls nach Entfernung der verwendeten niedrig siedenden organischen Lösungsmittel mit der Gießlösung für die lichtempfindliche Silberhalogenidemulsionsschicht oder einer anderen wasserdurchlässigen Schicht vor dem Auftragen vermischt wird.

Als lichtempfindliche Silberhalogenidemulsionen eignen sich Emulsionen von Silberchlorid, Silberbromid oder Gemischen davon, evtl. mit einem geringen Gehalt an Silberiodid bis zu 10 mol-% in einem der üblicherweise verwendeten hydrophilen Bindmittel. Als Bindemittel für die fotografischen Schichten wird vorzugsweise Gelatine verwendet. Diese kann jedoch ganz oder teilweise durch andere natürliche oder synthetische Bindemittel ersetzt werden.

Die Emulsionen können in der üblichen Weise chemisch und spektral sensibilisiert sein, und die Emulsionsschichten wie auch andere nicht-lichtempfindliche Schichten können in der üblichen Weise mit bekannten Härtungsmitteln gehärtet sein.

Üblicherweise enthalten farbfotografische Aufzeichnungsmaterialen mindestens je eine Silberhalogenidemulsionsschicht für die Aufzeichnung von Licht der drei Spektralbereiche Rot, Grün und Blau. Zu diesem Zweck sind die lichtempfindlichen Schichten in bekannter Weise durch geeignete Sensibilisierungsfarbstoffe spektral sensibilisiert. Blauempfindliche Silberhalogenidemulsionsschichten müssen nicht notwendigerweise einen Spektralsensibilisator enthalten, da für die Aufzeichnung von blauem Licht in vielen Fällen die Eigenempfindlichkeit des Silberhalogenids ausreicht.

Jede der genannten lichtempfindlichen Schichten kann aus einer einzigen Schicht bestehen oder in bekannter Weise, z.B. bei der sogenannten Doppelschichtanordnung, auch zwei oder mehr Silberhalogenidemulsionsteilschichten umfassen (DE-C-1 121 470). Üblicherweise sind rotempfindliche Silberhalogenidemulsionsschichten dem Schichtträger näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtemfindliche gelbe Filterschicht befindet. Es sind aber auch andere Anordnungen denkbar. Zwischen Schichten unterschiedlicher Spektralempfindlichkeit ist in der Regel eine nicht lichtempfindliche Zwischenschicht angeordnet, die Mittel zur Unterbindung der Fehldiffusion von Entwickleroxidationsprodukten enthalten kann. Falls mehrere Silberhalogenidemulsionsschichten gleicher Spektralempfindlichkeit vorhanden sind, können

diese einander unmittelbar benachbart sein oder so angeordnet sein, daß sich zwischen ihnen eine lichtempfindliche Schicht mit anderer Spektralempfindlichkeit befindet (DE-A-1 958 709, DE-A-25 30 645, DE-A-26 22 922).

Farbfotografische Aufzeichnungsmaterialien zur Herstellung mehrfarbiger Bilder enthalten üblicherweise in räumlicher und spektraler Zuordnung zu den Silberhalogenidemulsionsschichten unterschiedlicher Spektralempfindlichkeit farbgebende Verbindungen, hier besonders Farbkuppler, zur Erzeugung der unterschiedlichen Teilfarbenbilder Blaugrün, Purpur und Gelb.

Unter räumlicher Zuordnung ist dabei zu verstehen, daß der Farbkuppler sich in einer solchen räumlichen Beziehung zu der Silberhalogenidemulsionsschicht befindet, daß eine Wechselwirkung zwischen ihnen möglich ist, die eine bildgemäße Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit jeder der lichtempfindlichen Silberhalogendemulsionsschichten und die Farbe des aus dem jeweils räumlich zugeordneten Farbkuppler erzeugten Teilfarbenbildes in einer bestimmten Beziehung zueinander stehen, wobei jeder der Spektralempfindlichkeiten (Rot, Grün, Blau) eine andere Farbe des betreffenden Teilfarbenbildes (z.B. Blaugrün, Purpur, Gelb) zugeordnet ist.

Jeder der unterschiedlich spektral sensibilisierten Silberhalogenidemulsionsschichten kann ein oder können auch mehrere Farbkuppler zugeordnet sein. Wenn mehrere Silberhalogenidemulsionsschichten gleicher Spektralempfindlichkeit vorhanden sind, kann jede von ihnen einen Farbkuppler enthalten, wobei diese Farbkuppler nicht notwendigerweise identisch zu sein brauchen. Sie sollen lediglich bei der Farbentwicklung wenigstens annähernd die gleiche Farbe ergeben, normalerweise eine Farbe, die komplementär ist zu der Farbe des Lichtes, für das die betreffenden Silberhalogenidemulsionsschichten überwiegend empfindlich sind.

Rotempfindlichen Silberhalogenidemulsionsschichten ist folglich bei bevorzugten Ausführungsformen mindestens ein nichtdiffundierender Farbkuppler zur Erzeugung des blaugrünen Teilfarbenbildes zugeordnet, in der Regel ein Kuppler vom Phenol- oder α-Naphtholtyp. Vorteilhafte Blaugrünkuppler sind beispielsweise beschrieben in EP-A-0 028 099, EP-A-0 067 689, EP-A-0 175 573 und EP-A-0 184 057. Grünempfindlichen Silberhalogenidemulsionsschichten ist mindestens ein nichtdiffundierender Farbkuppler zur Erzeugung des purpurnen Teilfarbenbildes zugeordnet, wobei üblicherweise Farbkuppler vom Typ des 5-Pyrazolons, des Indazolons oder des Pyrazoloazols Verwendung finden. Blauempfindlichen Silberhalogenidemulsionsschichten schließlich ist mindestens ein nichtdiffundierender Farbkuppler zur Erzeugung des gelben Teilfarbenbildes zugeordnet, in der Regel ein Farbkuppler mit einer offenkettigen Ketomethylengruppierung. Farbkuppler dieser Art sind in großer Zahl bekannt und in einer Vielzahl von Patentschriften beschrieben. Beispielhaft sei hier auf die Veröffentlichungen "Farbkuppler" von W. PELZ in "Mitteilungen aus den Forschungslaboratorien der Agfa, Leverkusen/München", Band III, Seite 111 (1961) und von K. VENKATARAMAN in "The Chemistry of Synthetic Dyes", Vol. 4, 341 bis 387, Academic Press (1971), verwiesen.

Bei den Farbkupplern kann es sich sowohl um übliche 4-Äquivalentkuppler handeln als auch um 2-Äquivalentkuppler, bei denen zur Farberzeugung eine geringere Menge Silber halogenid erforderlich ist. 2-Äquivalentkuppler leiten sich bekanntlich von den 4-Äquivalentkupplern dadurch ab, daß sie in der Kupplungsstelle einen Substituenten enthalten, der bei der Kupplung abgespalten wird. Zu den 2-Äquivalentkupplern sind sowohl solche zu rechnen, die praktisch farblos sind, als auch solche, die eine intensive Eigenfarbe aufweisen, die bei der Farbkupplung verschwindet bzw. durch die Farbe des erzeugten Bildfarbstoffes ersetzt wird. Letztere Kuppler können ebenfalls zusätzlich in den lichtempfindlichen Silberhalogenidemulsionsschichten vorhanden sein und dort als Maskenkuppler zur Kompensierung der unerwünschten Nebendichten der Bildfarbstoffe dienen. Zu den 2-Äquivalentkupplern sind aber auch die bekannten Weißkuppler zu rechnen, die jedoch bei Reaktion mit Farbentwickleroxidationsprodukten keinen Farbstoff ergeben. Zu den 2-Äquivalentkupplern sind ferner die bekannten DIR-Kuppler zu rechnen, bei denen es sich um Kuppler handelt, die in der Kupplungsstelle einen abspaltbaren Rest enthalten, der bei Reaktion mit Farbentwickleroxidationsprodukten als diffundierender Entwicklungsinhibitor in Freiheit gesetzt wird. Auch andere fotografisch wirksame Verbindungen, z.B. Entwicklungsbeschleuniger oder Schleiermittel, können bei der Entwicklung aus solchen Kupplern freigesetzt werden.

Erfindungsgemäß enthält das farbfotografische Aufzeichnungsmaterial zusätzlich mindestens einen 2-Äquivalentgelbkuppler der Formel I, wobei dieser Kuppler nicht nur in der Gelbschicht, sondern auch in der Purpurschicht und/oder auch in der Blaugrünschicht sowie auch in einer zu einer der genannten Schichten benachbarten nicht lichtempfindlichen Schicht enthalten sein kann.

Über die genannten Bestandteile hinaus kann das farbfotografische Aufzeichnungsmaterial der vorliegenden Erfindung weitere Zusätze enthalten, zum Beispiel Antioxidantien, farbstoffstabilisierende Mittel und Mittel zur Beeinflussung der mechanischen und elektrostatischen Eigenschaften. Um die nachteilige Einwirkung von UV-Licht auf die mit dem erfindungsgemäßen farbfotografischen Aufzeichnungsmaterial hergestellten Farbbilder zu vermindern oder zu vermeiden, ist es vorteilhaft, in einer oder mehreren der in dem Aufzeichnungsmaterial enthaltenen Schichten, vorzugsweise in einer der oberen Schichten, UV-absorbierende Verbindungen zu verwenden. Geeignete UV-Absorber sind beispielsweise in US-A-3 253 921, DE-C-20 36 719 und EP-A-0 057 160 beschrieben.

Für die erfindungsgemäßen Materialien können die üblichen Schichtträger verwendet werden, siehe Research Disclosure Nr. 17 643, Abschnitt XVII.

Als Schutzkolloid bzw. Bindemittel für die Schichten des Aufzeichnungsmaterials sind die üblichen hydrophilen filmbildenden Mittel geeignet, z.B. Proteine, insbesondere Gelatine. Begußhilfsmittel und Weichmacher können verwendet werden. Verwiesen wird auf die in der Research Disclosure Nr. 17 643 in Abschnitt IX, XI und XII angegebenen Verbindungen.

Die Schichten des fotografischen Materials können in der üblichen Weise gehärtet sein, beispielsweise mit Härtern des Epoxidtyps, des heterocyclischen Ethylenimins und des Acryloyltyps, Weiterhin ist es auch möglich, die Schichten gemäß dem Verfahren der DE-A-22 18 009 zu härten, um farbfotografische Materialien zu erzielen, die für eine Hochtemperaturverarbeitung geeignet sind. Ferner ist es möglich, die fotografischen Schichten mit Härtern der Diazin-, Triazin- oder 1,2-Dihydrochinolin-Reihe zu härten oder mit Härtern vom Vinylsulfon-Typ. Weitere geeignete Härtungsmittel sind aus den DE-A-24 39 551, DE-A-22 25 230, DE-A-23 17 672 und aus der oben angegebenen Research Disclosure 17 643, Abschnitt XI bekannt.

Weitere geeignete Zusätze werden in der Research Disclosure 17 643 und in "Product Licensing Index" von Dezember 1971, Seiten 107-110, angegeben.

Zur Herstellung farbfotografischer Bilder wird das erfindungsgemäße farbfotografische Aufzeichnungsmaterial mit einer Farbentwicklerverbindung entwickelt. Als Farbentwicklerverbindung lassen sich sämtliche Entwicklerverbindungen verwenden, die die Fähigkeit besitzen, in Form ihres Oxidationsproduktes mit Farbkupplern zu Azomethinfarbstoffen zu reagieren. Geeignete Farbentwicklerverbindungen sind aromatische mindestens eine primäre Aminogruppe enthaltende Verbindungen vom p-Phenylendiamintyp, beispielsweise N,N-Dialkyl-p-phenylendiamine, wie N,N-Diethyl-p-phenylendiamin, 1-(N-ethyl-N-methylsulfonamidoethyl)-3-methyl-p-phenylendiamin, 1-(N-ethyl-N-hydroxyethyl-3-methyl-p-phenylendiamin und 1-(N-ethyl-N-methoxyethyl)-3-methyl-p-phenylendiamin.

Weitere brauchbare Farbentwickler sind beispielsweise beschrieben in J. Amer. Chem. Soc. 73, 3100 (1951) und in G. Haist, Modern Photographic Processing, 1979, John Wiley and Sons, New York, Seiten 545 ff.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren insbesondere z.B. Ethylendiamintetraessigsäure, N-Hydroxyethylethylendiamintriessigsäure, Alkyliminodicarbonsäuren und von entsprechenden Phosphonsäuren. Geeignet als Bleichmittel sind weiterhin Persulfate.

Beispiel 1

Ein farbfotografisches Aufzeichnungsmaterial für die Colornegativentwicklung wurde hergestellt, indem auf einen transparenten Schichtträger aus Cellulosetriacetat die folgenden Schichten in der angegebenen Reihenfolge aufgetragen wurden. Die Mengenangaben beziehen sich jeweils auf 1 $m^2$. Für den Silberhalogenidauftrag werden die entsprechenden Mengen $AgNO_3$ angegeben. Alle Silberhalogenidemulsionen waren pro 100 g $AgNO_3$ mit 0,5 g 4-Hydroxy-6-methyl-1,3,3a,7-tetraazainden stabilisiert.

Schicht 1

(Antihaloschicht)
Schwarzes kolloidales Silbersol mit 0,4 g
Ag und 3 g Gelatine.

Schicht 2

(Zwischenschicht)
0,5 g Gelatine

Schicht 3

(1. rotsensibilisierte Schicht)
rotsensibilisierte Silberbromidiodidemulsion (5 ml-% Iodid;
mittlerer Korndurchmesser 0,5 µm) aus
3,5 g $AgNO_3$, mit
0,7 g Kuppler C-1
0,06 g Maskenkuppler MC-1

DIR-Kuppler wie in Tabelle 1 angegeben
und
1,5 g Gelatine

Schicht 4

(2. rotsensibilisierte Schicht)
rotsensibilisierte Silberbromidiodidemulsion (10 mol-% Iodid;
mittlerer Korndurchmesser 1,5 µm) aus
3,7 g $AgNO_3$, mit
0,2 g Kuppler C-1 und
1,9 g Gelatine

Schicht 5

(Zwischenschicht)
0,8 g Gelatine

Schicht 6

(1. grünsensibilisierte Schicht)
grünsensibilisierte Silberbromidiodidemulsion (5 ml-% Iodid;
mittlerer Korndurchmesser 0,5 µm)
aus 2,5 g $AgNO_3$, mit
0,6 g Kuppler M-1
0,07 g Maskenkuppler MC-2,
DIR-Kuppler wie in Tabelle 1 angegeben
und
1,4 g Gelatine

Schicht 7

(2. grünsensibilisierte Schicht)
grünsensibilisierte Silberbromidiodidemulsion (10 mol-% Iodid;
mittlerer Korndurchmesser 1,4 µm) aus
2,1 g $AgNO_3$, mit
0,15 g Kuppler M-1
0,03 g Maskenkuppler MC-2 und
1,6 g Gelatine

Schicht 8

(Gelbfilterschicht)
gelbes kolloidales Silbersol mit 71 mg
Ag und 0,5 g Gelatine.

Schicht 9

(1. blauempfindliche Schicht)
Silberbromidiodidemulsion
(5 mol-% Iodid;
mittlerer Korndurchmesser 0,7 µm) aus
0,5 g $AgNO_3$,
mit 0,6 g Kuppler Y-1,
$4{,}2\times10^{-4}$ mol DIR-Kuppler DV-1 und
1,4 g Gelatine

Schicht 10

(2. blauempfindliche Schicht) Silberbromidiodidemulsion
(9 mol-% Iodid;
mittlerer Korndurchmesser 1,4 µm)
aus 1,5 g $AgNO_3$, mit
0,15 g Kuppler Y-1 und
0,7 g Gelatine

Schicht 11

(Schutzschicht)
0,7 g Gelatine

Schicht 12

(Härtungsschicht)
0,24 g Gelatine und 0,7 g Carbamoylpyridiniumsalz (CAS Reg. No. 65411-60-1)

Folgende Kuppler wurden verwendet:

Kuppler C-1

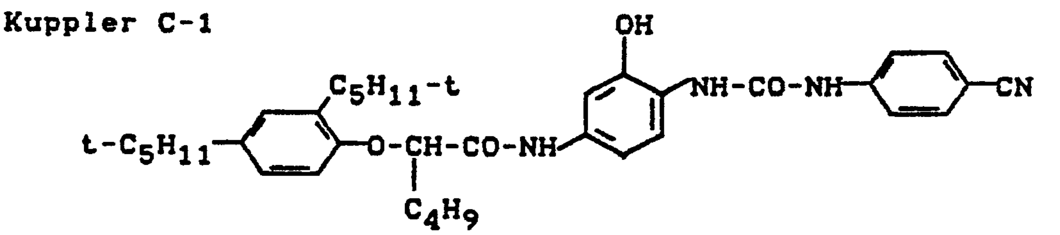

Kuppler M-1

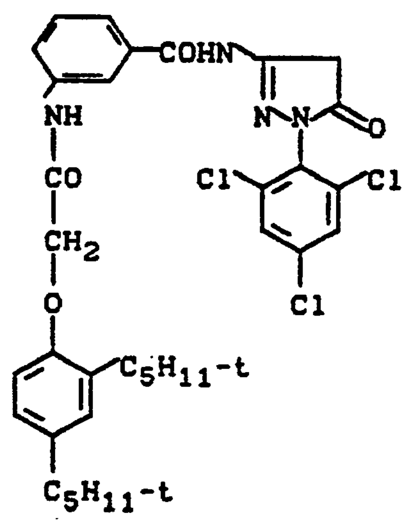

Maskenkuppler MC-1

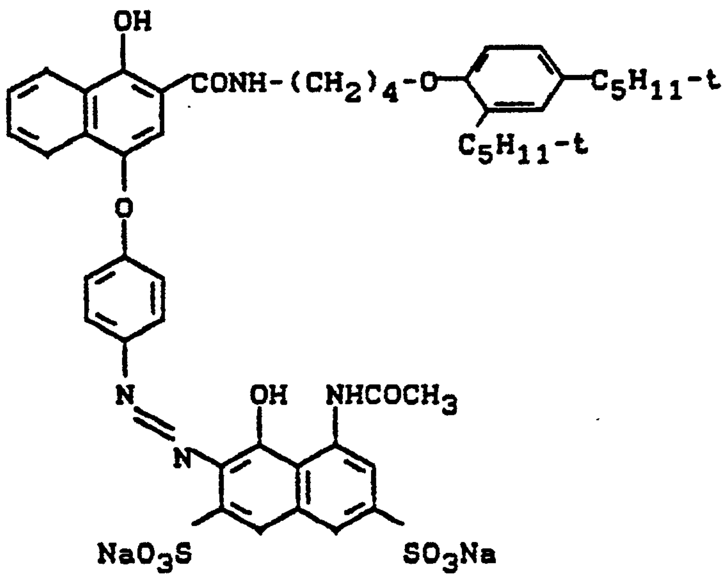

Maskenkuppler MC-2

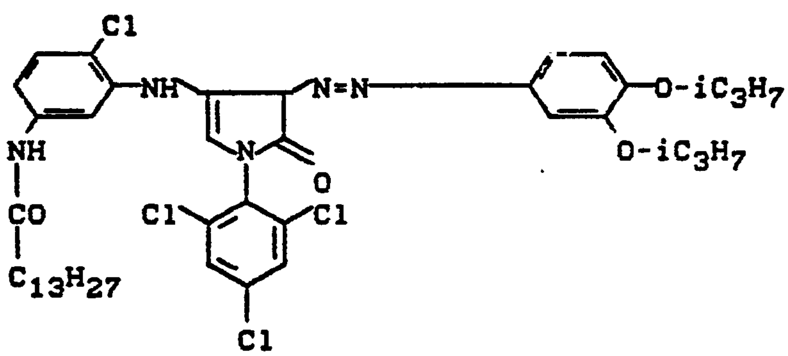

Kuppler Y-1

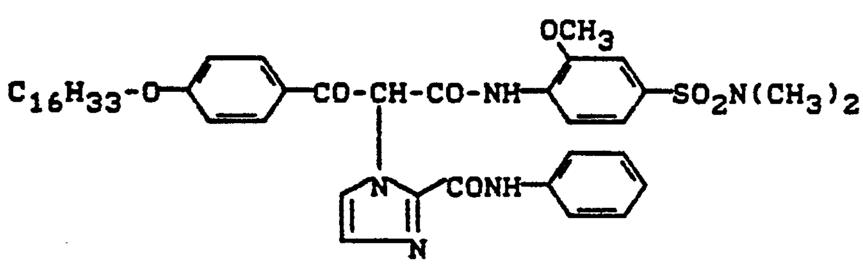

DIR-Kuppler DV-1

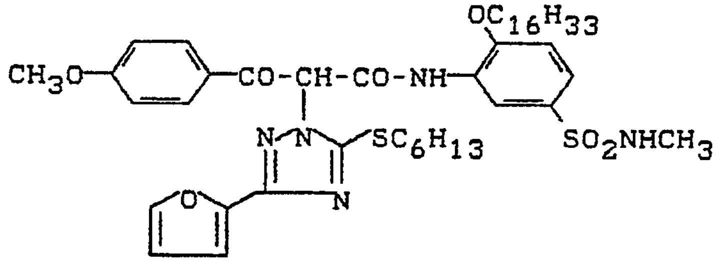

Die Verbindungen C-1, MC-2, Y-1 sowie die DIR-Kuppler wurden als Emulgate eingesetzt, wobei bezogen auf ein Teil der eingesetzten Verbindung 1 Teil Gelatine, 2 Teile Trikresylphosphat im Falle der Verbindungen M-1 und MC-2 bzw. Di-n-butylphthalat in allen anderen Fällen, und 0,1 Teile Na-Salz der Triisopropylnaphtalinsulfonsäure als Netzmittel verwendet wurden.

Von dem Aufzeichnungsmaterial des beschriebenen Aufbaus wurden verschiedene Versionen (Materialien 1-7) hergestellt, die sich ausschließlich durch den in den Schichten 3 und 6 verwendeten DIR-Kuppler unterscheiden. Die Entwicklung wurde nach Aufbelichtung eines Graukeils (hinter Farbfiltern) durchgeführt wie beschrieben in "The Journal of Photography, 1974, Seiten 597 und 598.

Die Ergebnisse nach Verarbeitung sind in Tabelle 1 dargestellt. Die Interimageeffekte IIE berechnen sich wie folgt:

$$IIE_{bg} = \frac{\gamma_{rot} - \gamma_w}{\gamma_w} \quad ; \quad IIE_{pp} = \frac{\gamma_{grün} - \gamma_w}{\gamma_w}$$

Dabei bedeutet:

$\gamma_{rot}$ Gradation bei selektiver Belichtung mit rotem Licht
$\gamma_{grün}$ Gradation bei selektiver Belichtung mit grünem Licht
$\gamma_w$ Gradation bei Belichtung mit weißem Licht
$KE_{bg}$ KE in der rotsensibilisierten Schicht
$KE_{pp}$ KE in der grünsensibilisierten Schicht

**Tabelle 1**

| Material | DIR-Kuppler [x $10^{-5}$ mol] Schicht 3 | | Schicht 6 | | $KE_{bg}$ | $KE_{pp}$ | $IIE_{bg}$ | $IIE_{pp}$ |
|---|---|---|---|---|---|---|---|---|
| 1 (Vergleich) | DV-1 | 7,1 | DV-1 | 7,0 | 0,33 | 0,36 | 30 | 28 |
| 2 (gem. Erf.) | D-1 | 4,0 | D-1 | 4,1 | 0,41 | 0,38 | 41 | 40 |
| 3 " | D-2 | 5,1 | D-1 | 7,0 | 0,38 | 0,41 | 30 | 50 |
| 4 " | D-3 | 5,9 | D-3 | 5,0 | 0,40 | 0,36 | 40 | 35 |
| 5 " | D-6 | 5,1 | DV-1 | 7,0 | 0,38 | 0,36 | 42 | 46 |
| 6 " | D-7 | 5,5 | DV-1 | 7,0 | 0,52 | 0,40 | 50 | 50 |
| 7 " | D-8 | 5,1 | DV-1 | 7,0 | 0,42 | 0,38 | 38 | 40 |

**Patentansprüche**

1. Farbfotografisches Aufzeichnungsmaterial mit mindestens einer lichtempfindlichen Silberhalogenidemulsionsschicht und einem dieser zugeordneten Kuppler, der eine fotografisch wirksame Verbindung freizusetzen vermag, dadurch gekennzeichnet, daß der Kuppler der folgenden allgemeinen Formel I entspricht

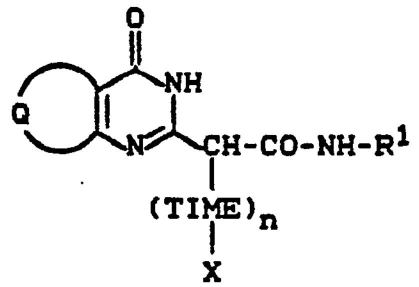

I

worin bedeuten:
$R^1$ Alkyl, eine heterocyclische oder carbocyclische aromatische Gruppe oder -NH-CO-$R^2$;
$R^2$ Alkyl, Aryl oder -NH-$R^3$;
$R^3$ Alkyl oder Aryl;
Q den Rest zur Vervollständigung eines ankondensierten, gegebenenfalls substituierten Benzol-oder heterocyclischen Ringes;
X den Rest einer fotografisch wirksamen Verbindung;
TIME ein Bindeglied, das bei Reaktion des Kupplers mit dem Oxidationsprodukt eines Farbentwicklers zusammen mit dem daran gebundenen Rest X freigesetzt wird und seinerseits unter den Entwicklungsbedingungen den Rest X als fotografisch wirksame Verbindung freisetzt;
n 0 oder 1.

2. Aufzeichnungsmaterial nach Anspruch 1, dadurch gekennzeichnet, daß der Kuppler ein DIR-Kuppler ist (X ist der Rest eines Entwicklungsinhibitors).

3. Aufzeichnungsmaterial nach einem der Ansprüche 1 und 2, dadurch gekennzeichnet, daß $R^1$ Phenyl bedeutet, das mit einem diffusionsfestmachenden Rest in Form einer Alkoxy-, Alkoxycarbonyl-, Carbamoyl-, Sulfamoyl, Sulfonamido- oder Carbonamidogruppe substituiert ist.

4. Aufzeichnungsmaterial nach einem der Ansprüche 1 und 2, dadurch gekennzeichnet, daß X für eine Gruppe der folgenden Formel

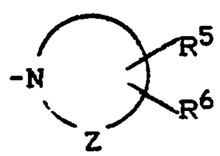

steht, worin bedeuten:
Z den Rest zur Vervollständigung eines 1,2,3- oder 1,2,4-Triazolringes;
$R^5$ und $R^6$ H, Alkyl, Aryl, eine heterocyclische Gruppe, Alkoxy, -S-$R^7$, Amino, Acylamino, eine Carbonsäureestergrupe oder -CO-$NR^8R^9$, oder $R^5$ und $R^6$ bedeuten zusammen (falls der durch Z vervollständigte Ring ein 1,2,3-Triazolring ist) den Rest zur Vervollständigung eines ankondensierten Ringes;
$R^7$ Alkyl, Cycloalkyl, Aralkyl, Alkenyl, Alkinyl oder Aryl;
$R^8$ Alkyl, Aralkyl oder Aryl;
$R^9$ H, einen Rest wie $R^8$
oder $R^8$ und $R^9$ zusammen den Rest zur Vervollständigung einer cyclischen Aminogruppe.

5. Aufzeichnungsmaterial nach Anspruch 2, dadurch gekennzeichnet, daß der DIR-Kuppler in einer überwiegend blauempfindlichen Silberhalogenidemulsionsschicht enthalten ist, und daß das Aufzeichnungsmaterial mindestens eine weitere überwiegend grünempfindliche oder überwiegend rotempfindliche Silberhalogenidemulsionsschicht enthält.

6. Aufzeichnungsmaterial nach Anspruch 2, dadurch gekennzeichnet, daß der DIR-Kuppler in einer überwiegend rotempfindlichen Silberhalogenidemulsionsschicht enthalten ist.

7. Farbfotografisches Aufzeichnungsmaterial mit mindestens einer überwiegend blauempfindlichen Silberhalogenidemulsionsschichteneinheit, der mindestens ein Gelbkuppler zugeordnet ist, einer überwiegend grünempfindlichen Silberhalogenidemulsionsschichteneinheit, der mindestens ein Purpurkuppler zugeordnet ist, und einer überwiegend rotempfindlichen Silberhalogenidemulsionsschichteneinheit, der mindestens ein Blaugrünkuppler zugeordnet ist, dadurch gekennzeichnet, daß mindestens eine Teilschicht der überwiegend grünempfindlichen Silberhalogenidemulsionsschichteneinheit oder der überwiegend rotempfindlichen Silberhalogenidemulsionschichteneinheit einen DIR-Kuppler der folgenden Formel I enthält:

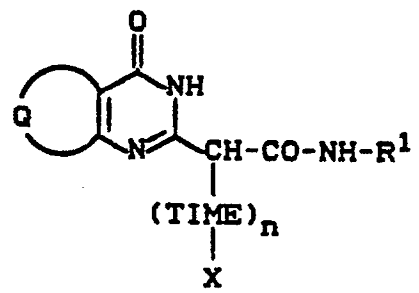

I

worin bedeuten:
$R^1$ Alkyl, eine heterocyclische oder carbocyclische aromatische Gruppe oder -NH-CO-$R^2$;
$R^2$ Alkyl, Aryl oder -NH-$R^3$;
$R^3$ Alkyl oder Aryl;
Q den Rest zur Vervollständigung eines ankondensierten, gegebenenfalls substituierten Benzol-oder heterocyclischen Ringes;
X den Rest ein Entwicklungsinhibitors;
TIME ein Bindeglied, das bei Reaktion des Kupplers mit dem Oxidationsprodukt eines Farbentwicklers zusammen mit dem daran gebundenen Rest X freigesetzt wird und seinerseits unter den Entwicklungsbedingungen den Rest X als Entwicklungsinhibitor freisetzt;
n 0 oder 1.

8. Verbindung der folgenden Formel

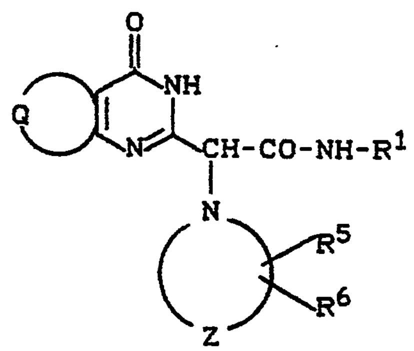

I

worin bedeuten:
$R^1$ Phenyl, das mit mindestens einer Alkoxy-, Alkoxycarbonyl-, Carbamoyl-, Sulfamoyl-, Sulfonamido- oder Carbonamidogruppe mit jeweils mindestens 8 C-Atomen substituiert ist;
Q den Rest zur Vervollständigung eines ankondensierten Benzolringes;
Z den Rest zur Vervollständigung eines 1,2,3-oder 1,2,4-Triazolringes;
$R^5$ und $R^6$ H, Alkyl, Aryl, eine heterocyclische Gruppe, Alkoxy, -S-$R^7$, Amino, Acylamino, eine Carbonsäureestergruppe oder -CO-$NR^8R^9$, oder $R^5$ und $R^6$ bedeuten zusammen (falls der durch Z vervollständigte Ring ein 1,2,3-Triazolring ist) den Rest zur Vervollständigung eines ankondensierten, gegebenenfalls substituierten Benzolringes
$R^7$ Alkyl, Cycloalkyl, Aralkyl, Alkenyl, Alkinyl oder Aryl;
$R^8$ Alkyl, Aralkyl oder Aryl;
$R^9$ H, einen Rest wie $R^8$
oder $R^8$ und $R^9$ zusammen den Rest zur Vervollständigung einer cyclischen Aminogruppe.

## Claims

1. A color photographic recording material comprising at least one photosensitive silver halide emulsion layer and, associated therewith, a coupler which is capable of releasing a photographically active compound, characterized in that the coupler corresponds to the following general formula

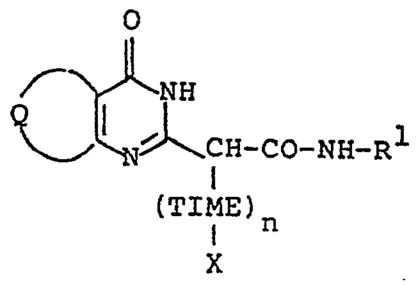

(I)

in which

$R^1$ represents alkyl, a heterocyclic or carbocyclic aromatic group or

–NH-CO-$R^2$;

$R^2$ represents alkyl, aryl or –NH-$R^3$;
R3 represents alkyl or aryl;
Q represents the residue required to complete a fused, optionally substituted benzene or heterocyclic ring;
X represents the residue of a photographically active compound;
TIME represents a bond which, on reaction of the coupler with the oxidation product of a color developer, is released together with the residue X attached thereto and, in turn, releases the residue X as a photographically active compound under the development conditions;
n = 0 or 1.

2. A recording material as claimed in claim 1, characterized in that the coupler is a DIR coupler (X is the residue of a development inhibitor).

3. A recording material as claimed in any of claims 1 and 2, characterized in that $R^1$ is a phenyl radical substituted by a group which imparts resistance to diffusion in the form of an alkoxy, alkoxycarbonyl, carbamoyl, sulfamoyl, sulfonamido or carbonamido group.

4. A recording material as claimed in claim 1 or 2, characterized in that X represents a group corresponding to the following formula

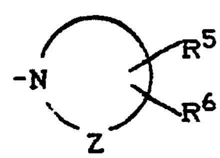

in which

Z represents the balance required to complete a 1, 2, 3 or 1, 2, 4-triazole ring;
$R^5$ and $R^6$ represent H, alkyl, aryl, a heterocyclic group, alkoxy, –S-$R^7$, amino, acylamino, a carboxylic acid ester group or –CO-$NR^8NR^9$, or $R^5$ and $R^6$ together represent (where the ring completed by Z is a 1, 2, 3-triazole ring) the balance required to complete a fused ring:
$R^7$ represents an alkyl, cycloalkyl, aralkyl, alkenyl, alkynyl or aryl;
$R^8$ represents alkyl, aralkyl or aryl;
$R^9$ represents H or has the same meaning as $R^8$ or $R^8$ and $R^9$ together represent the balance required to complete a cyclic amino group.

5. A recording material as claimed in claim 2, characterized in that the DIR coupler is present in a predominantly blue-sensitive silver halide emulsion layer and in that the recording material contains at least one other predominantly green-sensitive or predominantly red-sensitive silver halide emulsion layer.

6. A recording material as claimed in claim 2, characterized in that the DIR coupler is present in a predominantly red-sensitive silver halide emulsion layer.

7. A color photographic recording material comprising at least one predominantly blue-sensitive silver halide emulsion layer unit with which at least one yellow coupler is associated, a predominantly green-sensitive silver halide emulsion layer unit with which at least one magenta coupler is associated and a predominantly red-sensitive silver halide emulsion layer unit with which at least one cyan coupler is associated, characterized in that at least a partial layer of the predominantly green-sensitive silver halide emulsion layer unit or of the predominantly red-sensitive silver halide emulsion layer unit contains a DIR coupler corresponding to the following formula:

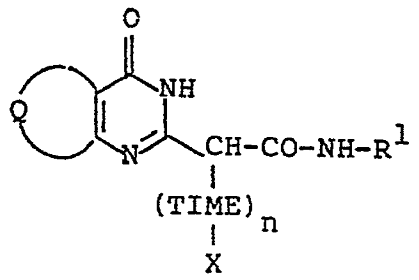

(I)

in which
$R^1$ represents alkyl, a heterocyclic or carbocyclic aromatic group or

–NH-CO-$R^2$;

$R^2$ represents alkyl, aryl or –NH-$R^3$;
$R^3$ represents alkyl or aryl;
Q represents the balance required to complete a condensed, optionally substituted benzene or heterocyclic ring;
X represents the residue of a development inhibitor;
TIME is a bond which, on reaction of the coupler with the oxidation product of a color developer, is released together with the residue X attached thereto and, in turn, releases the residue X with delay as a development in hibitor under the development conditions;
n = 0 or 1.

8. A compound corresponding to the following formula

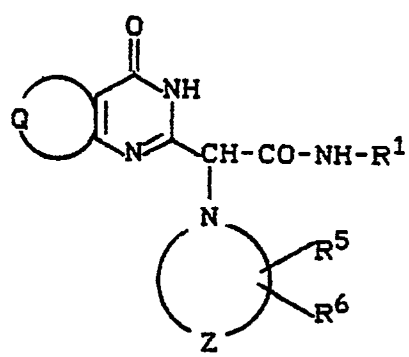

I

in which
$R^1$ represents phenyl substituted by at least one alkoxy, alkoxycarbonyl, carbamoyl, sulfamoyl, sulfonamido or carbonamido group each containing at least 8 carbon atoms;
Q represents the balance required to complete a fused benzene ring;
Z represents the balance required to complete a 1, 2, 3or 1, 2, 4-triazole ring:
$R^5$ and $R^6$ represent H, alkyl, aryl, a heterocyclic group, alkoxy, –S-$R^7$, amino, acylamino, a carboxylic ester group or –CO-$NR^8R^9$ or $R^5$ and $R^6$ together represent (where the ring completed by Z is a 1, 2, 3-triazole ring) the balance required to complete a fused, optionally substituted benzene ring;
$R^7$ represents alkyl, cycloalkyl, aralkyl, alkenyl, alkynyl or aryl:
$R^8$ represents alkyl, aralkyl or aryl;
$R^9$ represents H or has the same meaning as $R^8$ or $R^8$ and $R^9$ together represent the balance required to complete a cyclic amino group.

## Revendications

1. Matériau d'enregistrement chromophotographique muni d'au moins une couche d'émulsion à l'halogénure d'argent photosensible et d'un copulant qui lui est attribué, ce copulant étant susceptible de libérer un composé photographiquement actif, caractérisé en ce que le copulant répond à la formule I ci-après

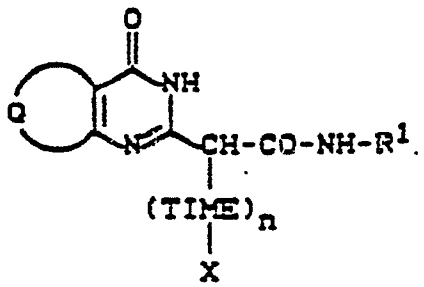

dans laquelle :
$R^1$ représente un groupe alkyle, un groupe aromatique hétérocyclique ou carbocyclique ou –NH-CO-$R^2$;
$R^2$ représente un groupe alkyle, un groupe aryle ou –NH-$R^3$;
$R^3$ représente un groupe alkyle ou un groupe aryle;
Q représente le radical destiné à compléter un noyau accolé hétérocyclique ou benzénique éventuellement substitué;
X représente le radical d'un composé photographiquement actif;
TIME représente un chaînon qui se libère, lors de la réaction du copulant avec le produit d'oxydation d'un révélateur chromogène, conjointement avec le radical X qui lui est lié et libère, à son tour, dans les conditions de développement, le radical X comme composé photographiquement actif;
n est égal à 0 ou 1.

2. Matériau d'enregistrement selon la revendication 1, caractérisé en ce que le copulant est un copulant DIR (X étant le radical d'un inhibiteur de développement).

3. Matériau d'enregistrement selon l'une quelconque des revendication 1 ou 2, caractérisé en ce que $R^1$ représente un groupe phényle qui est substitué par un radical non diffusible, par exemple sous forme d'un groupe alcoxy, alcoxycarbonyle, carbamoyle, sulfamoyle, sulfonamido ou carbonamido

4. Matériau d'enregistrement selon l'une quelconque des revendications 1 ou 2, caractérisé en ce que X représente un groupe de la formule ci-après,

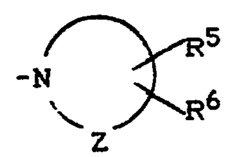

dans laquelle:
Z représente le radical destiné à compléter un noyau 1, 2, 3- ou 1, 2, 4-triazole i
$R^5$ et $R^6$ désignent un atome d'hydrogène, un groupe alkyle, un groupe aryle, un groupe hétérocyclique, un groupe alcoxy, –S-$R^7$, un groupe amino, un groupe acylamino, un groupe ester d'acide carboxylique ou –CO-$NS^8R^9$, ou bien $R^5$ et $R^6$ représentent ensemble (si le noyau complété par Z est un noyau 1, 2, 3-triazole) le radical destiné à compléter un noyau accolé;
$R^7$ représente un groupe alkyle, un groupe cycloalkyle, un groupe aralkyle, un groupe alcényle, un groupe alcynyle ou un groupe aryle;
$R^8$ représente un groupe alkyle, un groupe aralkyle ou un groupe aryle;
$R^9$ représente un atome d'hydrogène, un radical tel que $R^8$ ou $R^8$ et $R^9$ ensemble représentent le radical destiné à compléter un groupe amino cyclique.

5. Matériau d'enregistrement selon la revendication 2, caractérise en ce que le copulant DIR est contenu dans une couche d'émulsion à l'halogénure d'argent à sensibilité prédominante pour le bleu, et en ce que le matériau d'enregistrement contient au moins une autre couche d'émulsion à l'halogénure d'argent à sensibilité prédominante pour le vert ou à sensibilité prédominante pour le rouge.

6. Matériau d'enregistrement selon la revendication 2, caractérise en ce que le copulant DIR est contenu dans une couche d'émulsion à l'halogénure d'argent à sensibilité prédominante pour le rouge.

7. Matériau d'enregistrement chromophotographique muni d'au moins une unité de couche d'émulsion à l'halogénure d'argent à sensibilité prédominante pour le bleu, à laquelle est attribué au moins un copulant pour le jaune, une unité de couche d'émulsion à l'halogénure d'argent à sensibilité prédominante pour le

vert, à laquelle est attribué au moins un copulant pour le magenta, et une unité de couche d'émulsion à l'halogénure d'argent à sensibilité prédominante pour le rouge, à laquelle est attribué au moins un copulant pour le bleu-vert, caractérisé en ce qu'au moins une couche partielle de l'unité de couche d'émulsion à l'halogénure d'argent à sensibilité prédominante pour le vert ou de l'unité de couche d'émulsion à l'halogénure d'argent à sensibilité prédominante pour le rouge, contient un copulant DIR répondant à la formule I ci-après:

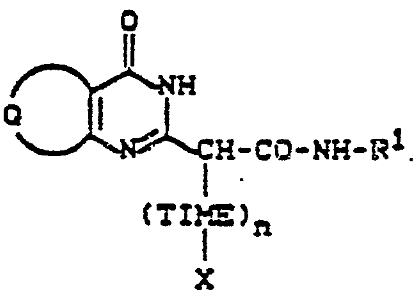

R1 représente un groupe alkyle, un groupe aromatique hétérocyclique ou carbocyclique ou –NH-CO-R2;
R2 représente un groupe alkyle, un groupe aryle ou –NH-R3;
R3 représente un groupe alkyle ou un groupe aryle;
Q représente le radical destiné à compléter un noyau accolé hétérocyclique ou benzénique éventuellement substitué;
X représente le radical d'un inhibiteur de développement
TIME représente un chaînon qui se libère, lors de la réaction du copulant avec le produit d'oxydation d'un révélateur chromogène, conjointement avec le radical X qui lui est lié et libère, à son tour, dans les conditions de développement, le radical X comme inhibiteur de développement;
n est égal à 0 ou 1.

8. Composé répondant à la formule ci-après:

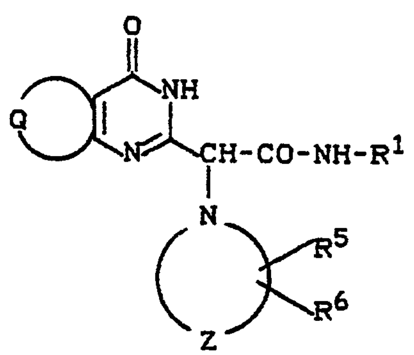

I

dans laquelle:
R1 représente un groupe phényle qui est substitué au moins par un groupe alcoxy, alcoxycarbonyle, carbamoyle, sulfamoyle, sulfonamido ou carbonamido, contenant chacun au moins 8 atomes de carbone;
Q représente le radical destiné à compléter un noyau benzénique accolé;
Z représente le radical destiné à compléter un noyau 1, 2, 3- ou 1, 2, 4-triazole;
R5 et R6 représentent un atome d'hydrogène, un groupe alkyle, un groupe aryle, un groupe hétérocyclique, un groupe alcoxy, $-S-R^7$, un groupe amino, un groupe acylamino, un groupe ester d'acide carboxylique ou $-CO-NR^8R^9$, ou bien $R^5$ et $R^6$ représentent ensemble (si le noyau complété par Z est un noyau 1, 2, 3-triazole) le radical destiné à compléter un noyau benzénique accolé éventuellement substitué;
$R^7$ représente un groupe alkyle, un groupe cycloalkyle, un groupe aralkyle, un groupe alcényle, un groupe alcynyle ou un groupe aryle;
$R^8$ représente un groupe alkyle, un groupe aralkyle ou un groupe aryle;
$R^9$ représente un atome d'hydrogène, un radical tel que $R^8$ou R8 et R9 ensemble représentent le radical destiné à compléter un groupe amino cyclique.